# EUROPÄISCHE PATENTSCHRIFT

(11) **EP 4 288 011 B1**
(45) Veröffentlichungstag und Bekanntmachung des Hinweises auf die Patenterteilung: **29.10.2025**
(21) Anmeldenummer: 22709597.3
(22) Anmeldetag: 01.02.2022
(51) Int. Cl.: A61F 13/532, A61F 13/534, A61F 13/84

(54) **INKONTINENZARTIKEL MIT TRANSFERKOMPONENTE**
INCONTINENCE ARTICLE WITH TRANSFER COMPONENT
ARTICLE D'INCONTINENCE AYANT UN COMPOSANT DE TRANSFERT

(30) Priorität: 05.02.2021 DE 102021102760
(43) Veröffentlichungstag der Anmeldung: 13.12.2023
(73) Patentinhaber: Paul Hartmann AG, 89522 Heidenheim (DE)
(72) Erfinder: FRÜHE, Thomas, 89518 Heidenheim (DE); FRANK, Bernd, 89555 Steinheim (DE); KNECHT, Theresia, 73433 Aalen (DE); PÉREZ ALONSO, Francisco José, 08120 Barcelona (ES); ESQUERRA, Juan, 08902 L Hospitalet de Llobregat, Barcelona (ES); MÉRIDA RIVERO, Juan José, 08924 Barcelona (ES); GRUESO, Daniel, 08820 El Prat de Llobregat (ES); FERNÁNDEZ GÁMIZ, Juan Francisco, 08830 Sant Boi de Llobregat Barcelona (ES); ARRANZ, David Olalla, 08021 Barcelona (ES)
(74) Vertreter: Paul Hartmann AG Patents & Licensing
(86) Internationale Anmeldenummer: PCT/EP2022/052318
(87) Internationale Veröffentlichungsnummer: WO 2022/167406

(56) Entgegenhaltungen:
- EP-A1- 3 838 243
- WO-A1-2019/167356
- WO-A2-2008/147991
- US-A1- 2006 206 077
- US-A1- 2012 232 510

## Beschreibung

Die vorliegende Erfindung betrifft einen Inkontinenzartikel für die Aufnahme von Körperausscheidungen mit einer Transferkomponente und pH-Regulationsmittel für die bevorzugte Verwendung durch Erwachsene.

Ein häufiges Problem bei der Verwendung von Inkontinenzartikeln ist das Auftreten von Hautreizungen oder Hautentzündungen, welche durch den Kontakt der Haut mit Körperausscheidungen wie beispielsweise Urin hervorgerufen werden. Oftmals kann sich eine sogenannte Inkontinenz-assoziierte Dermatitis (IAD) entwickeln, eine durch Kontakt mit einem Reizstoff wie Urin hervorgerufene Hautentzündung in der perinealen oder perigenitalen Region. Dabei spielen Veränderungen des pH-Wertes der Haut bei Kontakt mit dem alkalischeren Urin eine wesentliche Rolle. Um einen solchen Kontakt der Haut mit Urin zu verringern weisen Inkontinenzartikel typischerweise superabsorbierende Materialien auf, welche große Mengen an Urin aufnehmen und dauerhaft binden können. Außerdem kann mit einem entsprechenden Aufbau des Inkontinenzartikels, beispielsweise durch die Verwendung von Flüssigkeitsaufnahme- und Verteilerschichten im Inneren des Inkontinenzartikels, Flüssigkeiten schneller in den Inkontinenzartikel aufgenommen und dabei von der Haut des Benutzers weggeleitet werden. Teilweise verfügen Inkontinenzartikel auch über Öffnungen, Rillen oder Vertiefungen, welche zu einer besseren Flüssigkeitsleitung und -verteilung im Inkontinenzartikel beitragen sollen. Weiterhin werden häufig Substanzen in das absorbierende Material des Saugkörpers eingebracht, um den pH-Wert der aufgenommenen Körperflüssigkeiten zu regulieren. Dabei wird üblicherweise das absorbierende Material mit solchen Substanzen gleichmäßig vorgemischt, bevor aus der sich so ergebenden Mischung der Saugkörper ausgeformt wird. Das erfordert einen hohen Materialeinsatz bei den pH-regulierenden Substanzen, um eine gleichmäßige Einbringung und pH-Regulationswirkung zu ermöglichen. Gleichzeitig bleibt bei der Verwendung ein erheblicher Anteil der pH-regulierenden Substanzen ungenutzt, da Inkontinenzartikel typischerweise deutlich vor Erreichen der maximal möglichen Flüssigkeitsaufnahme des Saugkörpers ausgetauscht werden. Die Wirkung derart eingebrachter pH-regulierender Substanzen an einer Hautkontaktfläche des Inkontinenzartikels kann insbesondere bei Verwendung zusätzlicher Flüssigkeitsaufnahme- und Verteilerschichten oft nicht zufriedenstellend sichergestellt werden. Es besteht also weiterhin Bedarf an verbesserten Inkontinenzartikeln.

Inkontinenzartikel für Erwachsene sind seit langem bekannt und weisen häufig ein zumindest bereichsweise flüssigkeitsdurchlässiges Topsheet, ein im Wesentlichen flüssigkeitsundurchlässiges Backsheet und einen zwischen dem Topsheet und dem Backsheet angeordneten Absorptionskörper auf. Diese können grundsätzlich auf vielfältige Weise ausgestaltet sein, beispielsweise als direkt am Körper anliegende Inkontinenzprodukte oder als saugfähige Inkontinenzunterlagen.

Im Stand der Technik finden sich Ansätze, Inkontinenzartikel mit einem Mittel zur pH-Wert-Kontrolle bereitzustellen. WO2019167356A1 lehrt einen absorbierenden Artikel mit einem pH-einstellenden Mittel, welches am oder im absorbierenden Kern verortet ist. Die eigentliche pH-einstellende Substanz ist mit einer bei Raumtemperatur festen Oberflächenbeschichtung aus Öl oder Fett versehen, welche eine langsame Freisetzung der pH-einstellenden Substanz über eine längere Zeit hinweg bewirken soll. WO2008/147991A2 lehrt einen Polymer-Film, der ein Geruchskontrollmittel oder ein Hautpflegemittel wie beispielsweise ein pH-Regulationsmittel aufweist, und einen wegwerfbaren absorbierenden Artikel umfassend einen derartigen Polymer-Film. Der Polymer-Film soll durch Kontakt mit Körperflüssigkeit aktiviert werden, um das Geruchskontrollmittel oder das Hautpflegemittel freizusetzen. EP3838243A1 lehrt einen Inkontinenzartikel mit einem zwischen einem Topsheet und einem Backsheet angeordneten pH-Regulationsmittel, das Mononatriumcitrat oder Dinatriumcitrat aufweist. Das pH-Regulationsmittel kann zwischen einer Flüssigkeitsaufnahmeschicht und einer einem Backsheet zugewandten Absorptionskörperschicht angeordnet sein. WO2012121932A1 lehrt einen absorbierenden Artikel mit einer Pufferzusammensetzung, die mit absorbierendem Material vorgemischt ist oder die zwischen zwei absorbierenden Schichten des Saugkörpers angeordnet ist. Die Erfinder haben erkannt, dass eine solche Anordnung den Nachteil hat, dass die Körperflüssigkeit, die beim Eindringen in den Saugkörper in Kontakt mit der Pufferzusammensetzung kommt, diese beim Vordringen in tiefere Saugkörperlagen mit sich schwemmen kann oder überhaupt erst in tieferen Saugkörperlagen mit einer ausreichenden Menge der Pufferzusammensetzung in Kontakt kommt. Außerdem kann die Wirkung der Pufferzusammensetzung durch manche Komponenten des absorbierenden Materials wie z.B. superabsorbierende Polymere oder chemisch vorbehandelte Zellulosefasern beeinträchtigt werden.

Der vorliegenden Erfindung liegt daher die Aufgabe zu Grunde, diese Nachteile zu überwinden.

Diese Aufgabe wird gelöst durch einen Inkontinenzartikel für die Aufnahme von Körperausscheidungen, umfassend ein zumindest bereichsweise flüssigkeitsdurchlässiges Topsheet, ein im Wesentlichen flüssigkeitsundurchlässiges Backsheet und einen zwischen dem Topsheet und dem Backsheet angeordneten Absorptionskörper wobei der Absorptionskörper einen Speichersaugkörper und eine Transferkomponente aufweist, wobei die Transferkomponente zwischen dem Speichersaugkörper und dem Topsheet angeordnet ist, und wobei die Transferkomponente zumindest eine obere Transferlage und eine untere Transferlage aufweist, und wobei ein pH-Regulationsmittel zwischen der oberen Transferlage und der unteren Transferlage eingebracht ist.

Durch eine solche Anordnung des pH-Regulationsmittels wird in vorteilhafter Weise eine Fokussierung des pH-Regulationsmittels in der Nähe des Topsheets und damit eine effektive pH-Regulation nahe an einer hautseitigen Oberfläche des Inkontinenzartikels ermöglicht. Dadurch kann eine auf den Inkontinenzartikel auftreffende Körperflüssigkeit mit dem pH-Regulationsmittel in Kontakt treten, bevor sie in den Speichersaugkörper weitergeleitet und dauerhaft gebunden wird, wodurch die Wirkung des pH-Regulationsmittels beschleunigt werden kann. Außerdem wird die Gefahr der Beeinträchtigung der pH-Regulation durch Bestandteile des Speichersaugkörpers verringert.

Die Transferkomponente und die erste und zweite Transferlage sind nicht zu einer dauerhaften Speicherung von Körperflüssigkeiten, sondern zur raschen Aufnahme und unmittelbaren Weiterleitung von Körperflüssigkeiten an den Speichersaugkörper geeignet und bestimmt.

Dazu sind die obere und die untere Transferlage bevorzugt aus einem Fasermaterial, insbesondere einem Vliesmaterial ausgebildet und weisen weiter bevorzugt eine Dichte von 0,01 bis 0,10 g/cm³, insbesondere von 0,02 bis 0,08 g/cm³, weiter insbesondere von 0,03 bis 0,07 g/cm³ auf.

Bevorzugt sind die obere und die unteren Transferlage frei von superabsorbierendem Polymer (SAP) ausgebildet.

Weiter bevorzugt ist die Transferkomponente frei von SAP ausgebildet.

Die Fasern können natürlichen oder künstlichen Ursprungs sein und können von definierter Länge (Stapelfasern) oder kontinuierlich ("endlos") sein oder in-situ ausgebildet werden. Die Fasern können aus einem einzelnen Polymer oder einer Polymermischung (Einkomponentenfaser) oder aus mehr als einem einzelnen Polymer und/oder einer Polymermischung (Mehrkomponentenfaser) ausgebildet sein.

Eine Mehrkomponentenfaser weist einen Querschnitt auf, der mehr als einen einzelnen Abschnitt umfasst, wobei jeder dieser Abschnitte ein anderes Polymer oder eine andere Polymermischung umfasst. Der Begriff Mehrkomponentenfaser umfasst, ist aber nicht beschränkt auf, eine Zweikomponentenfaser. Die verschiedenen Komponenten von Mehrkomponentenfasern sind in im Wesentlichen unterschiedlichen Bereichen über den Querschnitt der Faser angeordnet und erstrecken sich kontinuierlich über die Länge der Faser. Eine Mehrkomponentenfaser kann einen Gesamtquerschnitt aufweisen, der Teilbereiche der zwei oder mehr unterschiedlichen Komponenten jeglicher Form oder Anordnung aufweist, einschließlich beispielsweise koaxialer Unterabschnitte, Kern- und Hüllenunterabschnitte, nebeneinander liegende Unterabschnitte, radiale Unterabschnitte, inselförmige Unterabschnitte, etc.

Eine Zweikomponentenfaser mit einer "Kern/Hüll-Struktur" hat einen Querschnitt, der Folgendes umfasst: zwei diskrete Abschnitte, von denen jeder aus einem Polymer oder einer Polymermischung besteht, worin das Hüllpolymer oder die Hüllpolymermischkomponente um das Kernpolymer oder die Kernpolymermischungskomponente herum angeordnet ist.

In einer bevorzugten Ausführungsform umfasst die Transferkomponente Mehrkomponentenfasern, insbesondere Zweikomponentenfasern, weiter insbesondere Polyester aufweisende Zweikomponentenfasern, sogenannte Bico/PES-Fasern. Die Mehrkomponentenfasern, insbesondere die Zweikomponentenfasern, weisen bevorzugt einen kreisförmigen oder dreilappigen Querschnitt auf.

Bevorzugte Kombinationen von Komponenten in Zweikomponentenfasern sind Polyethylenterephthalat (PET) / Polyethylen (PE), PET / Polypropylen (PP), PET / Polyester-Copolymere (CoPET), Polymilchsäure (PLA) / Polylactid-Copolymere (COPLA), PLA / PE und PLA / PP.

Die Transferkomponente kann auch andere Materialien wie perforierte Folien oder Schäume oder dergleichen umfassen oder daraus bestehen.

Die obere und die untere Transferlage können gleiche Materialien umfassen oder daraus bestehen oder unterschiedliche Materialien umfassen oder daraus bestehen.

Das Backsheet kann insbesondere ein atmungsaktives, jedoch im Wesentlichen flüssigkeitsdichtes Folienmaterial umfassen oder dadurch gebildet sein. Das Topsheet ist zumindest bereichsweise vorzugsweise von einem auf Vliesbasis beruhenden zumindest bereichsweise flüssigkeitsdurchlässigen Material gebildet.

In einer bevorzugten Ausführungsform unterscheidet sich die obere Transferlage hinsichtlich mindestens einer Eigenschaft ausgewählt aus der Gruppe Flächengewicht, Dichte, Reißfestigkeit, Opazität, Rücknässung, Flüssigkeitsdurchtrittszeit (Strike Through Time), Erstreckung in Längs- oder Quer- oder Dickenrichtung, Materialart, Faserart, Faserlänge, Faserdurchmesser, Faserkräuselung, Hydrophobizität, Art oder Menge an Additiven, von der unteren Transferlage.

Additive können beispielsweise Konfektionierungsmittel, Konditionierungsmittel, Netzmittel, Koagulationsmittel, Antikoagulationsmittel, adhäsive oder oberflächenaktive Substanzen oder antimikrobielle Substanzen, Farb- oder Duftstoffe, pH-Indikatoren oder hautpflegende Substanzen sein.

Die Dichte der oberen und unteren Transferlage wird wie dem Fachmann bekannt wie folgt berechnet: Dichte [g/cm³] = Flächengewicht [g/cm²] / Dicke [cm] Die Bestimmung des Flächengewichts erfolgt in einer dem Fachmann geläufigen Weise, indem die Masse eines typischerweise aus einem Flächenmaterial, beispielsweise einem Vliesmaterial, ausgestanzten Prüflings bestimmt und durch dessen Fläche geteilt wird. Bei der Ermittlung der Erstreckung in der Dickenrichtung, also einer Dicke, der oberen und unteren Transferlage, wird die jeweilige obere oder untere Transferlage unter einen Prüfdruck von 0,5 kPa gesetzt.

In einer bevorzugten Ausführungsform sind die obere Transferlage und die untere Transferlage durch eine, insbesondere in einer Längsrichtung verlaufende Falzkante miteinander verbunden.

Solchenfalls sind die obere und die untere Transferlage bevorzugt einstückig ausgebildet.

Alternativ können die obere und die untere Transferlage durch eine, insbesondere in einer Längsrichtung verlaufende Fügestelle miteinander verbunden sein. Das Fügen der oberen und unteren Transferlage kann hierbei mittels jedem dem Fachmann an sich bekannten und zu dem Zweck und entsprechend dem Material der oberen und unteren Transferlage geeigneten Fügeverfahren erfolgen, beispielsweise Thermoschweißen, Nähen, Siegeln, Kleben oder Ultraschallschweißen.

Als Längsrichtung ist im Rahmen dieser Erfindung eine Längsrichtung des Inkontinenzartikels zu verstehen, die sich in einem plan ausgebreiteten Zustand des Inkontinenzartikels von einer vorderen, also im Benutzungszustand bauchseitig zu liegen kommenden Querkante des Inkontinenzartikels zu einer hinteren, also im Benutzungszustand rückenseitig zu liegen kommenden Querkante des Inkontinenzartikels erstreckt. Die Längsrichtung des Inkontinenzartikels entspricht einer Längsrichtung der Transferkomponente und der oberen und unteren Transferlage und des Absorptionskörpers und des Speichersaugkörpers. Ebenso entspricht die Längsrichtung des Inkontinenzartikels gegebenenfalls einer Längsrichtung einer nachfolgend näher beschriebenen ersten und/oder zweiten Saugkörperlage.

Als eine Querrichtung ist weiterhin eine Querrichtung des Inkontinenzartikels zu verstehen, die orthogonal zur Längsrichtung verläuft. Die Querrichtung des Inkontinenzartikels entspricht einer Querrichtung der Transferkomponente und der oberen und unteren Transferlage und des Absorptionskörpers und des Speichersaugkörpers. Ebenso entspricht die Querrichtung des Inkontinenzartikels gegebenenfalls einer Querrichtung einer nachfolgend näher beschriebenen ersten und/oder zweiten Saugkörperlage.

Darüber hinaus weist der Inkontinenzartikel eine Dickenrichtung auf, die orthogonal zu der Längs- und zu der Querrichtung des Inkontinenzartikels verläuft. Die Dickenrichtung des Inkontinenzartikels entspricht einer Dickenrichtung der Transferkomponente und der oberen und unteren Transferlage und des Absorptionskörpers und des Speichersaugkörpers. Ebenso entspricht die Dickenrichtung des Inkontinenzartikels gegebenenfalls einer Dickenrichtung einer nachfolgend näher beschriebenen ersten und/oder zweiten Saugkörperlage.

In einer bevorzugten Ausführungsform ist das pH-Regulationsmittel gleichförmig verteilt zwischen der oberen und der unteren Transferlage eingebracht.

Hierbei ist zum Verständnis des Begriffs "gleichförmig" auf die Verteilung des pH-Regulationsmittels in einer die Längs- und die Querrichtung der oberen und unteren Transferlage umschließenden Ebene, also einer parallel zu einer der jeweiligen anderen oberen oder unteren Transferlage zugewandten Oberfläche der oberen oder unteren Transferlage angeordneten Ebene, abzustellen.

Durch eine solche Verteilung wird eine Gefahr einer ungleichmäßigen pH-Regulation verringert.

Außerdem verringert eine solche Anordnung zwischen der oberen und unteren Transferlage die Gefahr einer unerwünschten oder vorzeitigen Migration des pH-Regulationsmittels innerhalb des Inkontinenzartikels, insbesondere in den Speichersaugkörper hinein.

Der Speichersaugkörper des Inkontinenzartikels ist geeignet und dazu bestimmt, Körperausscheidungen, insbesondere Körperflüssigkeiten, insbesondere Urin aufzunehmen und im Speichersaugkörper dauerhaft zu speichern.

Dazu weist der Speichersaugkörper bevorzugt eine erste Saugkörperlage auf, wobei die erste Saugkörperlage weiter bevorzugt SAP aufweist.

In einer vorteilhaften Ausführungsform ist das SAP in der ersten Saugkörperlage gleichförmig verteilt eingebracht insbesondere derart, dass die Konzentration des SAP in Gewichtsprozent in einem mittleren Längsabschnitt der ersten Saugkörperlage gleich groß ist wie in einem vorderen und/oder einem hinteren Längsabschnitt der ersten Saugkörperlage.

Hierdurch ergeben sich Vorteile bei der Herstellung, da beispielsweise das SAP mit dem weiteren Material der ersten Saugkörperlage gleichförmig vorgemischt werden kann und eine schwierigere separate Dosierung und/oder Anordnung des SAP bei hoher Maschinengeschwindigkeit entfallen kann.

Alternativ kann das SAP auch ungleichförmig verteilt in der ersten Saugkörperlage eingebracht sein derart, dass die Konzentration des SAP in Gewichtsprozent in dem mittleren Längsabschnitt der ersten Saugkörperlage höher oder niedriger ist als in dem vorderen und/oder dem hinteren Längsabschnitt der ersten Saugkörperlage.

Dadurch ist es möglich, die flächenbezogene Menge des SAP in g/m² an die in dem jeweiligen mittleren oder vorderen oder hinteren Längsabschnitt der ersten Saugkörperlage benötigte Flüssigkeitsspeicherleistung anzupassen.

In einer bevorzugten Ausführungsform enthält die erste Saugkörperlage superabsorbierendes Polymer (SAP) zu 5 - 100 Gewichtsprozent, vorzugsweise zu 10 - 95 Gewichtsprozent, weiter vorzugsweise zu 15 - 90 Gewichtsprozent, ganz besonders bevorzugt zu 20 - 80 Gewichtsprozent. Typischerweise kann das SAP-Material zumindest das 15-fache, insbesondere das 20-fache seines Gewichts an 0,9 gewichtsprozentiger Kochsalzlösung absorbieren (gemessen nach NWSP 242.0.R2(15)).

Das SAP kann beispielsweise partikelförmig oder faserförmig oder blatt- oder schaumförmig ausgebildet sein.

Das SAP kann weiterhin ein im Wesentlichen sortenreines Polymermaterial oder eine Mischung von zwei oder mehreren Polymermaterialien umfassen oder daraus bestehen.

Die erste Saugkörperlage kann weitere Materialien, wie Zellstofffasern ("fluff") oder Kunststofffasern enthalten.

Solchenfalls ist das SAP vorzugsweise mit den weiteren Materialien, insbesondere Fasermaterialien, im Wesentlichen homogen vermischt, derart, dass das SAP gleichförmig verteilt entlang der Längs- und Querrichtung der ersten Saugkörperlage angeordnet ist. Bevorzugt ist das SAP auch gleichförmig verteilt entlang der Dickenrichtung der ersten Saugkörperlage angeordnet.

Denkbar ist weiterhin, die erste und/oder die nachfolgend näher beschriebene zweite Saugkörperlage des Speichersaugkörpers durch Anordnung von ein oder mehreren Schichten verschiedenen Materials (Materialschichten), insbesondere aus Vliesmaterial auszubilden.

Solchenfalls kann das SAP in Längs- und/oder Querrichtung gleichförmig verteilt auf einer Oberfläche mindestens einer der ersten und der nachfolgend näher beschriebenen zweiten Saugkörperlage oder einer Materialschicht oder zwischen der ersten und der zweiten Saugkörperlage oder zwischen zwei Materialschichten angeordnet sein.

Ein mittlerer Längsabschnitt der ersten Saugkörperlage ist einen Punkt oder Bereich des Auftreffens von Urin bei der Benutzung (Miktionsbereich) überfangend angeordnet und erstreckt sich über eine Quermittelachse der ersten Saugkörperlage hinweg. Der vordere Längsabschnitt der ersten Saugkörperlage schließt sich in Längsrichtung unmittelbar an den mittleren Längsabschnitt in Richtung einer vorderen, also im Benutzungszustand bauchseitig zu liegen kommenden Querkante der ersten Saugkörperlage an und erstreckt sich bis zu der vorderen Querkante der ersten Saugkörperlage.

Der hintere Längsabschnitt der ersten Saugkörperlage schließt sich in Längsrichtung unmittelbar an den mittleren Längsabschnitt in Richtung einer hinteren Querkante der ersten Saugkörperlage an und erstreckt sich bis zu der hinteren Querkante der ersten Saugkörperlage. Die Grenzen zwischen mittlerem und vorderem Längsabschnitt und zwischen mittlerem und hinterem Längsabschnitt verlaufen jeweils geradlinig in Querrichtung der ersten Saugkörperlage und orthogonal zu einer Längsmittelachse der ersten Saugkörperlage.

Der mittlere Längsabschnitt der ersten Saugkörperlage ist hinsichtlich seiner Längserstreckung symmetrisch zu der Quermittelachse der ersten Saugkörperlage angeordnet.

Eine Längserstreckung des mittleren Längsabschnitts der ersten Saugkörperlage beträgt im Rahmen der vorliegenden Erfindung 40% einer Gesamtlängserstreckung der ersten Saugkörperlage. Der hintere Längsabschnitt und der vordere Längsabschnitt der ersten Saugkörperlage betragen jeweils 30% einer Gesamtlängserstreckung der ersten Saugkörperlage.

Der mittlere, hintere und vordere Längsabschnitt der ersten Saugkörperlage erstreckt sich in Querrichtung der ersten Saugkörperlage über eine gesamte Breite der ersten Saugkörperlage an der jeweiligen Stelle, mithin von einer ersten Längskante der ersten Saugkörperlage bis zu einer zweiten Längskante der ersten Saugkörperlage.

Als Längserstreckung ist zu verstehen eine maximale Längserstreckung eines Elementes oder Längsabschnitts oder Bereichs.

Als Quererstreckung ist zu verstehen eine maximale Quererstreckung eines Elementes oder Längsabschnitts oder Bereichs.

Die erste Saugkörperlage kann rechteckig, dreieckig, oval, T-förmig, sanduhrförmig, anatomisch, asymmetrisch oder in einer anderen dem Fachmann als geeignet erscheinenden Form ausgebildet sein.

In einer bevorzugten Ausführungsform ist die flächenbezogene Menge des pH-Regulationsmittels in einem mittleren Längsabschnitt des Absorptionskörpers in g/m² größer ist als in einem vorderen und/oder einem hinteren Längsabschnitt des Absorptionskörpers.

Der mittlere Längsabschnitt des Absorptionskörpers ist hierbei in einem Schrittbereich des Inkontinenzartikels, mithin den Punkt oder Bereich des Auftreffens von Urin bei der Benutzung (Miktionsbereich) überfangend angeordnet und erstreckt sich über eine Quermittelachse des Absorptionskörpers hinweg. Der vordere Längsabschnitt des Absorptionskörpers schließt sich in Längsrichtung unmittelbar an den mittleren Längsabschnitt in Richtung einer vorderen, also im Benutzungszustand bauchseitig zu liegen kommenden Querkante des Absorptionskörpers an und erstreckt sich bis zu der vorderen Querkante des Absorptionskörpers.

Der hintere Längsabschnitt des Absorptionskörpers schließt sich in Längsrichtung unmittelbar an den mittleren Längsabschnitt in Richtung einer hinteren, also im Benutzungszustand rückenseitig zu liegen kommenden Querkante des Absorptionskörpers an und erstreckt sich bis zu der hinteren Querkante des Absorptionskörpers. Die Grenzen zwischen mittlerem und vorderem Längsabschnitt und zwischen mittlerem und hinterem Längsabschnitt verlaufen jeweils geradlinig in Querrichtung des Inkontinenzartikels und orthogonal zu einer Längsmittelachse des Absorptionskörpers.

Der mittlere Längsabschnitt des Absorptionskörpers ist hinsichtlich seiner Längserstreckung symmetrisch zu der Quermittelachse des Absorptionskörpers angeordnet.

Eine Längserstreckung des mittleren Längsabschnitts des Absorptionskörpers beträgt im Rahmen der vorliegenden Erfindung 40% einer Gesamtlängserstreckung des Absorptionskörpers. Der hintere Längsabschnitt und der vordere Längsabschnitt des Absorptionskörpers betragen jeweils 30% einer Gesamtlängserstreckung des Absorptionskörpers.

Der mittlere, hintere und vordere Längsabschnitt des Absorptionskörpers erstreckt sich in Querrichtung des Absorptionskörpers über eine gesamte Breite des Absorptionskörpers, mithin von einer ersten Längskante des Absorptionskörpers bis zu einer zweiten Längskante des Absorptionskörpers.

Als Längserstreckung ist zu verstehen eine maximale Längserstreckung eines Elementes oder Längsabschnitts oder Bereichs.

Als Quererstreckung ist zu verstehen eine maximale Quererstreckung eines Elementes oder Längsabschnitts oder Bereichs.

Der Absorptionskörper kann rechteckig, dreieckig, oval, T-förmig, sanduhrförmig, anatomisch, asymmetrisch oder in einer anderen dem Fachmann als geeignet erscheinenden Form ausgebildet sein.

Hierbei ist zu erwähnen, dass der Absorptionskörper und/oder die erste Saugkörperlage eine gleichförmige Dicke und/oder eine gleichförmige Dichte aufweisen kann. Ebenso ist denkbar, dass der Absorptionskörper und/oder die erste Saugkörperlage ein Dicken- oder Höhenprofil oder ein Dichteprofil aufweist.

In einem bevorzugten Ausführungsbeispiel ist 5,00-30,00 g/m², insbesondere 6,00-25,00 g/m², weiter insbesondere 7,00-20,00 g/m², weiter insbesondere 8,00-15,00 g/m² pH-Regulationsmittel im mittleren Längsabschnitt des Absorptionskörpers angeordnet.

Weiter bevorzugt sind der vordere und/oder hintere Längsabschnitt des Absorptionskörpers im Wesentlichen frei von pH-Regulationsmittel.

Es ist jedoch auch denkbar, dass der vordere und/oder der hintere Längsabschnitt des Absorptionskörpers pH-Regulationsmittel aufweisen. Solchenfalls kann im vorderen und/oder hinteren Längsabschnitt des Absorptionskörpers jeweils 0,00-10,00 g/m², insbesondere 0,10-9,00 g/m², weiter insbesondere 0,20-7,00 g/m², weiter insbesondere 0,30-5,00 g/m² pH-Regulationsmittel angeordnet sein.

In einer bevorzugten Ausführungsform weist die erste Saugkörperlage einen in der Längsrichtung orientierten Kanal auf.

Der Kanal erleichtert eine rasche Aufnahme von Körperflüssigkeiten wie Urin und dient deren anschließenden Weiterleitung in weiter entfernte Regionen des Absorptionskörpers.

Vorzugsweise überfängt die Transferkomponente den Kanal zumindest bereichsweise, insbesondere vollständig.

Bei dieser Anordnung können Körperflüssigkeiten wie Urin rasch aufgenommen, verteilt und in den Kanal weitergeleitet werden. Zudem können aufgenommene Körperflüssigkeiten in vorteilhafter Weise bereits beim Passieren der Transferkomponente mit dem pH-Regulationsmittel in Kontakt treten und der pH-Wert rasch reguliert werden. Dabei unterstützt der Kanal eine frühzeitige und gleichmäßige pH-Regulation durch das in der Transferkomponente eingebrachte pH-Regulationsmittel, bevor die Körperflüssigkeiten im Absorptionskörper und insbesondere durch SAP gebunden werden.

Bevorzugt ist der Kanal parallel zu einer Längsmittelachse des Absorptionskörpers und/oder der ersten Saugkörperlage und/oder des Inkontinenzartikels angeordnet.

Insbesondere ist der Kanal auf einer Längsmittelachse des Inkontinenzartikels und/oder des Absorptionskörpers und/oder der ersten Saugkörperlage angeordnet.

Weiter insbesondere ist der Kanal symmetrisch zur Längsmittelachse des Inkontinenzartikels und/oder des Absorptionskörpers und/oder der ersten Saugkörperlage ausgebildet.

Der Kanal ist auf vorteilhafte Weise in Querrichtung des Absorptionskörpers und/oder der ersten Saugkörperlage zentriert angeordnet.

Vorteilhafterweise ist der Kanal in Längsrichtung des Inkontinenzartikels im Bereich des Auftreffens von Urin bei der Benutzung angeordnet.

Weiterhin erstreckt sich der Kanal in vorteilhafter Weise über die Quermittelachse des Absorptionskörpers hinweg.

Ein bevorzugter Kanal ist derart angeordnet, dass er von der hinteren Querkante des Absorptionskörpers weiter entfernt ist als von der vorderen Querkante des Absorptionskörpers. Jedoch kann der Kanal auch gleich weit von der vorderen und hinteren Querkante des Absorptionskörpers entfernt oder weiter von der vorderen als von der hinteren Querkante des Absorptionskörpers entfernt angeordnet sein.

In einer bevorzugten Ausführungsform erstreckt sich der Kanal geradlinig in der Längsrichtung des Inkontinenzartikels und/oder des Absorptionskörpers und/oder der ersten Saugkörperlage. Dadurch kann die Flüssigkeit sich im Kanal ungehinderter in der Längsrichtung des Inkontinenzartikels ausbreiten.

Jedoch sind auch wellenförmige, gezackte oder bogenförmige oder jede andere Kanalform denkbar, die der Fachmann für sinnvoll erachtet.

Unterschiedliche Kanalformen wie beispielsweise rechteckig, mandelförmig, knochenförmig, T-förmig, dreieckig, oval, sternförmig oder verzweigt sind im Stand der Technik hinreichend bekannt.

Die Erfinder haben festgestellt, dass für eine rasche Flüssigkeitsleitung in Längsrichtung jedoch gerade solche Kanäle vorteilhaft sind, deren Erstreckung in der Längsrichtung des Inkontinenzartikels größer ist als deren Erstreckung in Querrichtung. Insbesondere rechteckige, mandelförmige oder ovale Kanäle haben sich als vorteilhaft erwiesen.

In einer bevorzugten Ausführungsform umfasst der Kanal eine sich durch die erste Saugkörperlage in Dickenrichtung hindurcherstreckende Ausnehmung oder ist daraus gebildet.

Der Kanal ist bevorzugt im Wesentlichen frei von absorbierenden Materialien.

Dazu kann der Kanal durch Weglassen des absorbierenden Materials oder durch Entfernen von absorbierendem Material mittels Schneiden oder Stanzen ausgebildet werden.

Ebenso denkbar ist jedoch, dass der Kanal durch Prägen oder Komprimieren der ersten Saugkörperlage ausgebildet wird.

In einer bevorzugten Ausführungsform ist der Kanal der ersten Saugkörperlage im Wesentlichen frei von SAP.

Das bietet den Vorteil, dass die in den Kanal aufgenommene Körperflüssigkeit nicht im Kanal vorzeitig dauerhaft gebunden wird, sondern ungehindert entlang des Kanals weitergeleitet und dadurch gleichmäßiger im Absorptionskörper verteilt werden kann. Insbesondere kann eine vorzeitige Bindung der aufgenommenen Körperflüssigkeit mittels SAP im Kanal die pH-Regulation der Körperflüssigkeit behindern oder zumindest verzögern.

Nach einer bevorzugten Ausführungsform weist die erste Saugkörperlage einen einzigen Kanal auf.

In einer alternativen Ausführungsform weist die erste Saugkörperlage mehr als einen Kanal, insbesondere 2-5, weiter insbesondere genau 2 Kanäle auf.

In einer bevorzugten Ausführungsform weist die erste Saugkörperlage einen ersten Bereich auf, wobei die erste Saugkörperlage in dem ersten Bereich SAP aufweist.

Weiter bevorzugt grenzt der erste Bereich an den Kanal an, insbesondere umgibt der erste Bereich den Kanal vollständig.

Das ermöglicht, dass der pH-Wert der in den Kanal aufgenommenen Körperflüssigkeit, insbesondere Urin, bereits vor einer dauerhaften Bindung durch SAP reguliert werden kann.

In einer weiteren bevorzugten Ausführungsform weist der Speichersaugkörper eine zweite Saugkörperlage auf, wobei die zweite Saugkörperlage unterhalb der ersten Saugkörperlage angeordnet ist, also zwischen der ersten Saugkörperlage und dem Backsheet.

Weiter bevorzugt weist die zweite Saugkörperlage weniger als 20 Gewichtsprozent, 15 Gewichtsprozent, 10 Gewichtsprozent, 5 Gewichtsprozent SAP auf, insbesondere ist die zweite Saugkörperlage frei von SAP.

Die zweite Saugkörperlage bewirkt auf vorteilhafte Weise einen höheren Tragekomfort, insbesondere bei einem am Körper anliegenden Inkontinenzartikel, da von der Backsheetseite her ein weicherer haptischer Eindruck des Inkontinenzartikels erreicht wird. Hierzu enthält die zweite Saugkörperlage wenig oder kein SAP. Dem Fachmann ist außerdem bekannt, dass partikelförmiges Material abrasive Kräfte ausüben kann, die das im Wesentlichen flüssigkeitsundurchlässige Backsheet, insbesondere eine flüssigkeitsundurchlässige Backsheetfolie des absorbierenden Artikels beschädigen und zu Undichtigkeit des Artikels führen können. Daher bietet die zweite Saugkörperlage auch den Vorteil, dass die Gefahr der Beschädigung der Backsheet-Folie im Herstellungsprozess oder während der Benutzung des Inkontinenzartikels verringert wird.

Es hat sich als vorteilhaft erwiesen, wenn das pH-Regulationsmittel partikelförmig ausgebildet ist.

Bevorzugt weist mindestens 50 Gewichtsprozent des pH-Regulationsmittels eine Partikelgröße von 10 bis 2000 µm, insbesondere von 50 bis 1200 µm, weiter insbesondere von 80 bis 800 µm auf.

Grundsätzlich sind auch pH-Regulationsmittel mit geringerer oder größerer Partikelgröße erhältlich und verwendbar.

Jedoch bieten die bevorzugten Partikelgrößen den Vorteil, dass sich Partikel einer solchen Größe bei Benetzung mit Körperausscheidungen wie einer Miktionsflüssigkeit langsamer auflösen als pH-Regulationsmittel geringerer Partikelgröße, so dass das pH-Regulationsmittel während der Benutzung nicht so schnell ausgewaschen wird und ein pH-regulierender Effekt an einer der Haut des Benutzers zugewandten Oberseite des Inkontinenzartikels länger erhalten bleibt.

Zudem lässt sich ein pH-Regulationsmittel der bevorzugten Partikelgröße insbesondere in schnell laufenden Maschinen leichter dosieren und in den Inkontinenzartikel, insbesondere in die Transferkomponente einarbeiten als beispielsweise feinpudrige pH-Regulationsmittel, da es im Herstellungsprozess zu weniger Staubentwicklung und damit verbundenem Materialverlust kommt.

Außerdem wird die prozesssichere Anordnung einer vorgesehenen Menge an pH-Regulationsmittel in einem vorgesehenen Zielbereich positiv beeinflusst.

Andererseits bieten Partikel dieser Größe auch den Vorteil, dass sie während der Benutzung des Inkontinenzartikels haptisch weniger wahrnehmbar sind als beispielsweise Granulate mit höherer Partikelgröße, wodurch der Tragekomfort des Inkontinenzartikels, insbesondere bei einer Anordnung in der Nähe einer hautseitigen Oberfläche des Inkontinenzartikels, verbessert wird.

Alternativ zu partikelförmigem pH-Regulationsmittel kann das pH-Regulationsmittel auch in gelöster Form, insbesondere als wässrige oder alkoholische Lösung in die Transferkomponente eingebracht sein, insbesondere durch Besprühen und optional nachfolgendem Trocknen mindestens einer der oberen oder unteren Transferlage. Als weitere Alternative kann das pH-Regulationsmittel auch als Suspension oder Paste in die Transferkomponente eingebracht sein.

Körperausscheidungen wie Urin weisen typischerweise einen alkalischeren pH-Wert als die Haut auf und können deshalb zu Hautirritationen oder -entzündungen führen. Daher sind im Stand der Technik pH-Regulationsmittel für Inkontinenzartikel zur pH-Kontrolle von Körperausscheidungen beschrieben wie schwache Säuren oder Basen und deren jeweilige Salze oder Kombinationen davon, beispielsweise Carbonsäuren wie Citronensäure, Essigsäure, Äpfelsäure, Milchsäure und/oder deren jeweilige Salze wie beispielsweise Natriumsalze. Grundsätzlich sind alle Substanzen oder Zusammensetzungen denkbar, die eine geeignete Pufferwirkung aufweisen und bei denen eine geringe Gefahr für Hautirritationen besteht. Hierbei gilt im Rahmen der vorliegenden Erfindung, dass superabsorbierende Polymere (SAP), also insbesondere Polymere, die zumindest das 15-fache ihres Gewichts an 0,9 gewichtsprozentiger Kochsalzlösung absorbieren (gemessen nach NWSP 242.0.R2(15)), nicht als pH-Regulationsmittel zu verstehen sind.

Die Erfinder haben festgestellt, dass es diesbezüglich vorteilhaft ist, wenn das pH-Regulationsmittel Trinatriumcitrat, Mononatriumcitrat oder Dinatriumcitrat aufweist. In einer bevorzugten Ausführungsform kann das pH-Regulationsmittel entweder Mononatriumcitrat und Trinatriumcitrat, oder Dinatriumcitrat und Trinatriumcitrat, oder Mononatriumcitrat und Dinatriumcitrat und Trinatriumcitrat aufweisen.

Durch die Auswahl oder Kombination der genannten Komponenten kann eine Feineinstellung der Pufferwirkung eines Inkontinenzartikels je nach angestrebter Benutzungsdauer oder Absorptionskapazität des Artikels erreicht werden, ohne dass wie im Falle der Verwendung von Citronensäure die Gefahr der Entstehung stark sauer reagierender Bereiche besteht.

Falls das pH-Regulationsmittel mehr als ein Citrat umfasst, weist das pH-Regulationsmittel vorzugsweise eine homogene Mischung der jeweiligen Citrate auf. Das hat den Vorteil, dass lokale Unterschiede in der Pufferwirkung des pH-Regulationsmittels vermieden werden können.

Die Begriffe Mononatriumcitrat, Dinatriumcitrat, und Trinatriumcitrat umfassen sowohl die nicht hydrierte Form als auch Hydrate der jeweiligen Substanz.

Bevorzugt besteht das pH-Regulationsmittel aus Mononatriumcitrat oder Dinatriumcitrat.

Ein Vorteil bei der Verwendung von Mononatriumcitrat ist beispielsweise, dass Mononatriumcitrat weniger hygroskopisch ist als beispielsweise Citronensäure und daher kann ein unerwünschter Eintrag von Feuchtigkeit in den Inkontinenzartikel vor der Benutzung vermieden werden. Da Mononatriumcitrat weniger hygroskopisch ist als beispielsweise Citronensäure ergibt sich als weiterer Vorteil eine bessere Rieselfähigkeit, so dass sich Mononatriumcitrat leichter dosieren und verteilen lässt.

In einer bevorzugten Ausführungsform besteht das pH-Regulationsmittel aus Mononatriumcitrat.

In einer weiteren bevorzugten Ausführungsform besteht das pH-Regulationsmittel aus Dinatriumcitrat.

Durch die Verwendung nur einer Komponente als pH-Regulationsmittel ergeben sich prozesstechnische Vorteile, da keine Vermischung verschiedener Komponenten oder Aufrechterhaltung einer homogenen Durchmischung während des Herstellungsprozesses erforderlich ist. Zudem wird eine gleichmäßige Pufferwirkung über die pH-Regulationsmittel enthaltenden Bereiche des Inkontinenzartikels hinweg weiter unterstützt.

In einer bevorzugten Ausführungsform beträgt die flächenbezogene Menge an pH-Regulationsmittel in der Transferkomponente 5 bis 100 g/m², insbesondere 7 bis 90 g/m², weiter insbesondere 10 bis 80 g/m².

Die Gesamtmenge an pH-Regulationsmittel pro Inkontinenzartikel, der insbesondere zur Benutzung durch Erwachsene vorgesehen ist, beträgt vorzugsweise 0,20 bis 3,00 g, weiter vorzugsweise 0,30 bis 2,50 g, weiter vorzugsweise 0,40 bis 2,00 g, weiter vorzugsweise 0,50 bis 1,50 g, weiter vorzugsweise 0,50 bis 1,00 g, weiter vorzugsweise 0,50 bis 0,80 g.

Die Mengenangaben des pH-Regulationsmittels sind so zu verstehen, dass sie sich auf ein wasserfreies pH-Regulationsmittel, beispielsweise nicht hydriertes Mononatriumcitrat, beziehen. Falls das pH-Regulationsmittel ein oder mehrere Hydrate oder wie nachfolgend beschrieben mehr als 5%, insbesondere mehr als 10% additive Substanzen umfasst, wird die Gesamtmenge des pH-Regulationsmittels in g/m² derart angepasst, dass die Gesamtmenge an dem enthaltenen reinen und/oder nicht hydrierten pH-Regulationsmittel der vorstehenden bevorzugten Menge entspricht.

Bevorzugt wird das Mononatriumcitrat oder Dinatriumcitrat oder Trinatriumcitrat im Wesentlichen in reiner Form verwendet, insbesondere mit einem Reinheitsgrad von mindestens 90%, weiter insbesondere mindestens 95%, weiter insbesondere mindestens 98%, weiter insbesondere mindestens 99%, weiter insbesondere 100%. Insbesondere wird das Mononatriumcitrat oder Dinatriumcitrat oder Trinatriumcitrat mit einer Reinheit verwendet, welche den Anforderungen der German Pharmaceutical Codex (DAC) oder der Commission Regulation (EU) 231/2012 genügt.

Dadurch können durch Rückstände anderer Substanzen ausgelöste Hautirritationen bei Benutzung des Inkontinenzartikels weitestgehend vermieden werden.

Es ist jedoch auch denkbar, dass das pH-Regulationsmittel eine geringe Menge einer oder mehrerer additiver Substanzen aufweist, beispielsweise Konfektionierungs- und/oder Konditionierungsmittel wie Trennmittel, Stabilisatoren, Staubbindemittel, Antibackmittel, Netzmittel, Koagulationsmittel, Antikoagulationsmittel, adhäsive oder oberflächenaktive Substanzen oder antimikrobielle Substanzen, Farb- oder Duftstoffe oder pH-Indikatoren. Solchenfalls beträgt der Anteil der einen oder mehreren anderen Substanzen an der Gesamtmenge des pH-Regulationsmittels insgesamt bevorzugt weniger als 10%, weiter bevorzugt weniger als 5%, weiter bevorzugt weniger als 2%, weiter bevorzugt weniger als 1%.

Die Transferkomponente kann die erste Saugkörperlage und/oder den Absorptionskörper in der Querrichtung im Wesentlichen vollständig oder nur teilweise überfangen.

Bevorzugt überfängt die Transferkomponente den Absorptionskörper und/oder die erste Saugkörperlage in Längsrichtung nur bereichsweise.

Mithin weist die Transferkomponente im Wesentlichen eine zumindest bereichsweise geringere flächenhafte Erstreckung als die erste Saugkörperlage und/oder der Absorptionskörper in Längs- und/oder Querrichtung des plan aufliegenden Inkontinenzartikels auf.

Bevorzugt überfängt die Transferkomponente die erste Saugkörperlage und/oder den Absorptionskörper zu 5-100%, insbesondere zu 10-90%, weiter insbesondere zu 15-80%, weiter insbesondere zu 15-70% ihrer flächenhaften Erstreckung.

Vorzugsweise ist die Transferkomponente zumindest in einem Bereich angeordnet, in welchem die Miktionsflüssigkeit in Gebrauch auf den Inkontinenzartikel trifft. Insbesondere erstreckt sich die Transferkomponente über und im Bereich einer Quermittelachse des Absorptionskörpers.

In einer bevorzugten Ausführungsform erhält der Inkontinenzartikel über mindestens zwei, insbesondere mindestens drei Miktionsereignisse hinweg, einen hautfreundlichen pH-Wert aufrecht.

Dadurch wird der Tragekomfort auch über einen längeren Benutzungszeitraum wie beispielsweise während der Nachtruhe verbessert.

Weiter bevorzugt beträgt der pH-Wert auf einer der Haut zugewandten Oberseite des Inkontinenzartikels einen Wert von 4,0-6,5, insbesondere 4,2-6,2, weiter insbesondere 4,3-6,0, weiter insbesondere 4,5-5,8, weiter insbesondere 4,7-5,7.

Bevorzugt weist die erste und/oder die zweite Saugkörperlage weniger als 20 Gewichtsprozent, insbesondere weniger als 15 Gewichtsprozent, weiter insbesondere weniger als 10 Gewichtsprozent, weiter insbesondere weniger als 5 Gewichtsprozent des pH-Regulationsmittels auf. Weiter insbesondere ist die erste und/oder die zweite Saugkörperlage frei von pH-Regulationsmittel.

Dadurch wird auf vorteilhafte Weise ein höherer Tragekomfort erreicht, insbesondere bei einem am Körper anliegenden Inkontinenzartikel, da von der Topsheetseite und/oder von der Backsheetseite her ein weicherer haptischer Eindruck des Inkontinenzartikels erreicht wird. Wie vorgehend beschrieben kann partikelförmiges Material abrasive Kräfte ausüben, die das im Wesentlichen flüssigkeitsundurchlässige Backsheet, insbesondere eine flüssigkeitsundurchlässige Backsheetfolie des absorbierenden Artikels beschädigen und zu Undichtigkeit des Artikels führen können. Daher ist ein weiterer Vorteil, dass die Gefahr der Beschädigung der Backsheet-Folie im Herstellungsprozess oder während der Benutzung des Inkontinenzartikels verringert wird.

Außerdem kann so in der Gebrauchssituation die Gefahr einer von einem Miktionsereignis unabhängigen unerwünschten pH-Wert-Absenkung an einer der Haut zugewandten oder an der Haut des Anwenders anliegenden Oberfläche des Inkontinenzartikels, beispielsweise wenn der Anwender des Inkontinenzartikels schwitzt, verringert werden.

In einer bevorzugten Ausführungsform ist das pH-Regulationsmittel zumindest teilweise und wenigstens an einer der oberen oder unteren Transferlage immobilisiert, insbesondere mittels Klebstoff immobilisiert.

Während des Herstellungs- und Verpackungsprozesses sowie insbesondere beim Anlegen und Tragen eines Inkontinenzartikels wirken mehrfach Kräfte auf den Inkontinenzartikel und/oder die Transferkomponente. Beispielsweise kann es beim Verpacken oder in der Benutzungssituation dazu kommen, dass die Transferkomponente komprimiert oder geknautscht wird. Durch eine zumindest teilweise oder vollständige Immobilisierung kann die Gefahr der Migration des pH-Regulationsmittels, beispielsweise die Ausbildung von lokalen Anhäufungen, verringert werden.

Bei dem Inkontinenzartikel kann es sich um unterschiedliche Ausführungsformen handeln wie beispielsweise Inkontinenzvorlagen, Inkontinenzeinlagen, Slipeinlagen, Inkontinenzwindeln offenen Typs, Inkontinenzwindeln geschlossenen Typs oder auch Krankenunterlagen.

Je nach Typ oder Ausführungsform kann der Inkontinenzartikel ein oder mehrere weitere im Stand der Technik hinlänglich bekannte Merkmale aufweisen wie beispielsweise Seitenteile, Verschlusssysteme, Flügel, Elastifizierungen, Auslaufschutzsysteme (z.B. Cuffs), Nässeanzeiger, Kennzeichnungsmittel, hautpflegende oder geruchsregulierende Substanzen oder Zusammensetzungen, Befestigungsmittel (z.B. Klebstoff, Haken) sowie andere dem Fachmann bekannte oder sinnvoll erscheinende Merkmale.

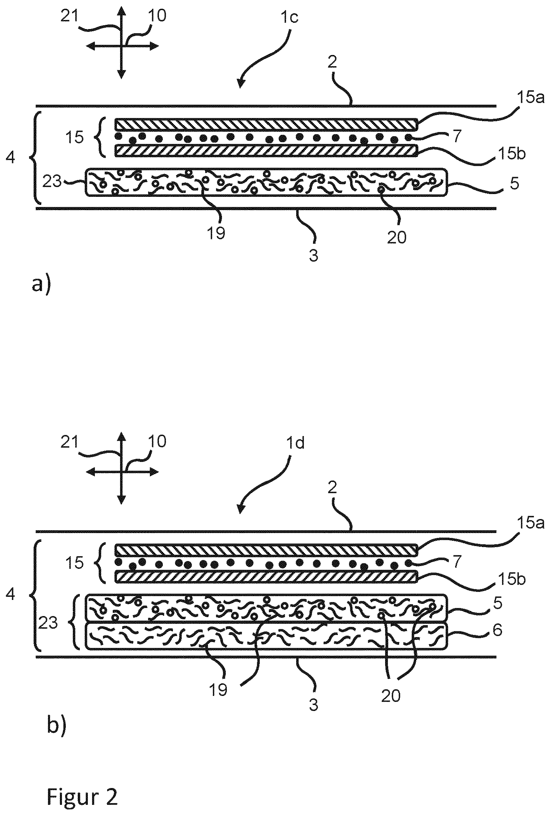

Weitere Merkmale, Einzelheiten und Vorteile der Erfindung ergeben sich aus den beigefügten Patentansprüchen und aus der zeichnerischen Darstellung und nachfolgenden Beschreibung bevorzugter Ausführungsformen der Erfindung und Beispielen. In der Zeichnung zeigt:
**Figur 1a**
   schematische Darstellung in Draufsicht eines Inkontinenzartikels mit Transferkomponente und pH-Regulationsmittel
**Figur 1b**
   schematische Darstellung in Draufsicht eines Inkontinenzartikels mit Transferkomponente und pH-Regulationsmittel sowie einem Kanal
**Figur 2a**
   schematisch, einen Querschnitt durch einen Inkontinenzartikel mit einer ersten Saugkörperlage, Transferkomponente und pH-Regulationsmittel
**Figur 2b**
   schematisch, einen Querschnitt durch einen Inkontinenzartikel mit einer ersten Saugkörperlage mit SAP und einer zweiten Saugkörperlage, einer Transferkomponente und pH-Regulationsmittel
**Figur 2c**
   schematisch, einen Querschnitt durch einen Inkontinenzartikel analog Figur 2a, jedoch mit einer einstückigen Transferkomponente
**Figur 2d**
   schematisch, einen Querschnitt durch einen Inkontinenzartikel analog Figur 2b, jedoch mit einer einstückigen Transferkomponente
**Figur 3a**
   schematisch, einen Querschnitt durch einen Inkontinenzartikel mit einer ersten Saugkörperlage, Transferkomponente, pH-Regulationsmittel und einem Kanal
**Figur 3b**
   schematisch, einen Querschnitt durch einen Inkontinenzartikel mit einer ersten Saugkörperlage mit SAP und einer zweiten Saugkörperlage, einer Transferkomponente, pH-Regulationsmittel und einem Kanal

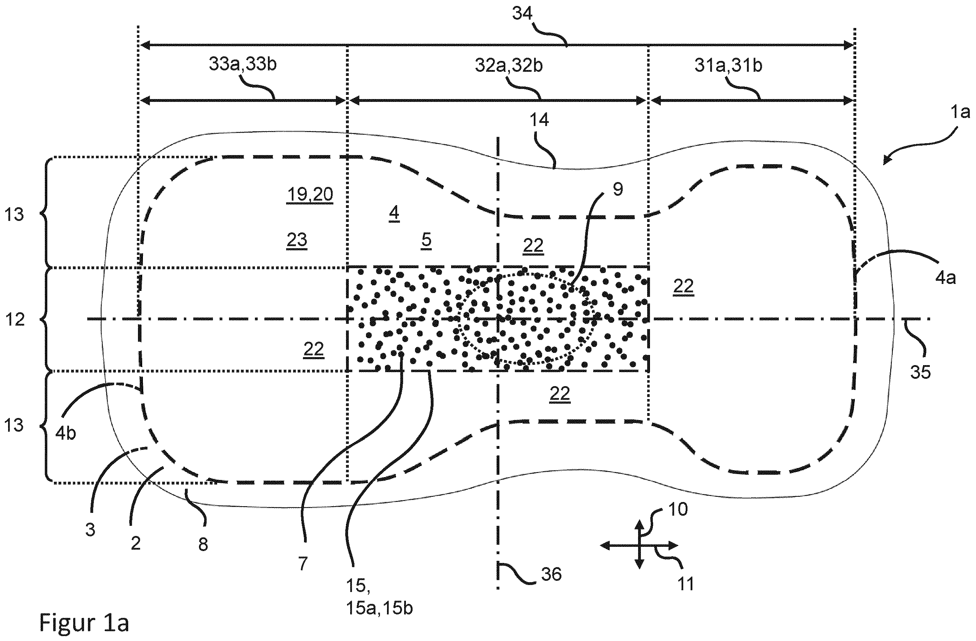

**Figur 1a** zeigt schematisch, nicht maßstäblich, einen erfindungsgemäßen Inkontinenzartikel 1a mit einem zumindest bereichsweise flüssigkeitsdurchlässigen Topsheet 2, einem im Wesentlichen flüssigkeitsundurchlässigen Backsheet 3 und einen zwischen dem Topsheet 2 und dem Backsheet 3 angeordneten Absorptionskörper 4, wobei der Absorptionskörper 4 eine Quermittelachse 36 und eine Längsmittelachse 35 aufweist.

Der Absorptionskörper 4 weist einen Speichersaugkörper 23 und eine Transferkomponente 15 auf, wobei die Transferkomponente 15 zwischen dem Speichersaugkörper 23 und dem Topsheet 2 angeordnet ist, und wobei die Transferkomponente 15 zumindest eine mit Bezug zu **Figur 2a****-d** näher beschriebene obere 15a und eine untere Transferlage 15b aufweist. Ein hier punktförmig dargestelltes pH-Regulationsmittel 7, beispielsweise partikelförmiges Mononatriumcitrat oder Trinatriumcitrat oder Dinatriumcitrat umfassend oder daraus bestehend oder eine Kombination davon, ist zwischen der oberen Transferlage 15a und der unteren Transferlage 15b angeordnet. Das pH-Regulationsmittel 7 ist gleichförmig verteilt zwischen der oberen Transferlage 15a und der unteren Transferlage 15b, nämlich in einer eine Längs- 11 und eine Querrichtung 10 der oberen 15a und unteren Transferlage 15b umschließenden Ebene angeordnet. In diesem bevorzugten Beispiel beträgt die flächenbezogene Menge an pH-Regulationsmittel 7 23 g/m².

Der Speichersaugkörper 23 weist eine erste Saugkörperlage 5 auf, die ein mit Bezug zu **Figur 2a****-d** näher beschriebenes SAP 20 und Fasermaterial 19 umfasst. Das SAP 20 ist in der ersten Saugkörperlage 5 gleichförmig verteilt angeordnet insbesondere derart, dass die Konzentration des SAP 20 in Gewichtsprozent in einem mittleren Längsabschnitt 32a der ersten Saugkörperlage 5 gleich groß ist wie in einem vorderen 31a und/oder einem hinteren Längsabschnitt 33a der ersten Saugkörperlage 5.

Die erste Saugkörperlage 5 weist einen ersten Bereich 22 auf und die erste Saugkörperlage 5 weist in dem ersten Bereich 22 SAP 20 auf.

In diesem Beispiel besteht der Speichersaugkörper 23 aus der ersten Saugkörperlage 5, so dass eine Erstreckung der ersten Saugkörperlage 5 in Längs- 11 und Querrichtung 10 einer jeweiligen Gesamterstreckung des Speichersaugkörpers 23 und einer jeweiligen Gesamterstreckung des Absorptionskörpers 4 entspricht. Ebenso entspricht hier auch der vordere Längsabschnitt 31a, der mittlere Längsabschnitt 32a und der hintere Längsabschnitt 33a der ersten Saugkörperlage 5 einem jeweiligen vorderen 31b, mittleren 32b und hinteren Längsabschnitt 33b des Absorptionskörpers 4.

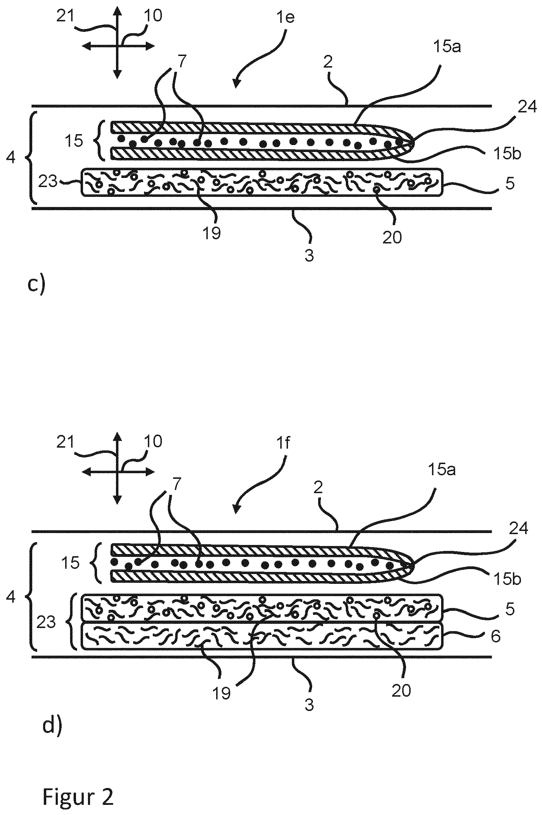

Optional kann der Speichersaugkörper 23 zusätzlich eine mit Bezug zu **Figur 2b** **und** **2d** näher beschriebene zweite Saugkörperlage 6 umfassen. Solchenfalls kann sich die Erstreckung der ersten Saugkörperlage 5 in der Längs- 11 und/oder Querrichtung 10 von einer Erstreckung der zweiten Saugkörperlage 6 und/oder von der Gesamterstreckung des Speichersaugkörpers 23 und/oder von der Gesamterstreckung des Absorptionskörpers 4 unterscheiden.

Der Absorptionskörper 4 weist eine im Benutzungszustand bauchseitig zu liegen kommende vordere Querkante 4a und eine im Benutzungszustand rückenseitig zu liegen kommende hintere Querkante 4b auf, die sich jeweils in einer Querrichtung 10 des Absorptionskörpers 4, welche hier einer Querrichtung des Inkontinenzartikels 1a und einer Querrichtung der ersten Saugkörperlage 5 entspricht, erstreckt. Die vordere 4a und die hintere Querkante 4b des Absorptionskörpers 4 verlaufen hier bereichsweise gerade. Alternativ können die vordere 4a und/oder die hintere Querkante 4b des Absorptionskörpers auch vollständig gerade, abgewinkelt, gekrümmt, gebogen, wellenförmig oder mit einer anderen dem Fachmann als geeignet erscheinenden Linienführung verlaufen.

In diesem Beispiel erstreckt sich die Transferkomponente 15 in Längsrichtung 11 über den gesamten mittleren Längsabschnitt der ersten Saugkörperlage 32a hinweg, weist also eine gleich große Längserstreckung auf wie der mittlere Längsabschnitt der ersten Saugkörperlage 32a. Es ist auch denkbar, dass die Transferkomponente 15 eine geringere oder größere Längserstreckung aufweist als der mittlere Längsabschnitt der ersten Saugkörperlage 32a und/oder in der Längsrichtung 11 in Richtung der vorderen Querkante 4a des Absorptionskörpers 4 oder der hinteren Querkante 4b des Absorptionskörpers 4 relativ zum mittleren Längsabschnitt der ersten Saugkörperlage 32a versetzt angeordnet ist.

Die Transferkomponente 15 und das pH-Regulationsmittel 7 sind in diesem Beispiel beidseitig der Längsmittelachse 35 des Absorptionskörpers 4, dabei in einem in Querrichtung 10 zentralen Bereich 12 des Absorptionskörpers 4 angeordnet, wobei in Querrichtung 10 periphere Bereiche 13 des Absorptionskörpers 4 frei von pH-Regulationsmittel 7 sind. Dadurch wird das pH-Regulationsmittel 7 dort fokussiert, wo Körperflüssigkeiten typischerweise vorrangig auf den Inkontinenzartikel 1a auftreffen, nämlich im zentralen Bereich 12. Die Transferkomponente 15 und das pH-Regulationsmittel 7 sind dabei auch in einem Miktionsbereich 9 (auch als Miktionspunkt bezeichnet) angeordnet, in welchem eine Miktionsflüssigkeit im Benutzungszustand mit hoher Wahrscheinlichkeit auf den Inkontinenzartikel 1a trifft. Es ist aber auch möglich, das pH-Regulationsmittel 7 sowohl im zentralen Bereich 12 als auch in den peripheren Bereichen 13 anzuordnen.

Die flächenbezogene Menge des pH-Regulationsmittels 7 ist in dem mittleren Längsabschnitt 32b des Absorptionskörpers 4 in g/m² größer als in dem vorderen 31b und dem hinteren Längsabschnitt 33b des Absorptionskörpers 4. In diesem Beispiel sind der vordere 31b und der hintere Längsabschnitt 33b des Absorptionskörpers 4 frei von pH-Regulationsmittel 7.

Der mittlere Längsabschnitt 32b des Absorptionskörpers 4 ist in einem Schrittbereich 14 des Inkontinenzartikels 1a, mithin sich über den Miktionsbereich 9 erstreckend, zu verorten und erstreckt sich zudem über die Quermittelachse 36 des Absorptionskörpers 4 hinweg. Der mittlere Längsabschnitt 33b des Absorptionskörpers 4 ist dabei symmetrisch zu der Quermittelachse 36 des Absorptionskörpers 4 angeordnet.

Der vordere Längsabschnitt 31b des Absorptionskörpers 4 schließt sich in Längsrichtung 11 an den mittleren Längsabschnitt 32b in Richtung der vorderen Querkante 4a des Absorptionskörpers 4 an und erstreckt sich bis zu der vorderen Querkante 4a des Absorptionskörpers 4.

Der hintere Längsabschnitt 33b des Absorptionskörpers 4 schließt sich in Längsrichtung 11 an den mittleren Längsabschnitt 32b in Richtung der hinteren Querkante 4b des Absorptionskörpers 4 an und erstreckt sich bis zu der hinteren Querkante 4b des Absorptionskörpers 4. Die Grenzen zwischen mittlerem 32b und vorderem Längsabschnitt 31b und zwischen mittlerem 32b und hinterem Längsabschnitt 33b verlaufen jeweils geradlinig in Querrichtung 10 des Inkontinenzartikels 1a und orthogonal zu der Längsmittelachse 35 des Absorptionskörpers 4.

In diesem bevorzugten Beispiel entspricht die Längsmittelachse 35 des Absorptionskörpers 4 einer Längsmittelachse des Inkontinenzartikels 1a und einer Längsmittelachse der ersten Saugkörperlage 5. Ebenso entspricht hier die Längsrichtung 11 des Absorptionskörpers 4 einer Längsrichtung des Inkontinenzartikels 1a und einer Längsrichtung der ersten Saugkörperlage 5.

Weiterhin entspricht die Quermittelachse 36 des Absorptionskörpers 4 einer Quermittelachse des Inkontinenzartikels 1a und einer Quermittelachse der ersten Saugkörperlage 5. Ebenso entspricht hier die Querrichtung 10 des Absorptionskörpers 4 einer Querrichtung des Inkontinenzartikels 1a und einer Querrichtung der ersten Saugkörperlage 5.

Die erste Saugkörperlage 5 ist in diesem bevorzugten Ausführungsbeispiel deckungsgleich, also in einer Längsrichtung 11 des Absorptionskörpers 4 und in einer Querrichtung 10 des Absorptionskörpers 4 gleich weit erstreckt wie der Absorptionskörper 4. Daher entspricht die Gesamtlängserstreckung 34 des Absorptionskörpers 4 einer Gesamtlängserstreckung der ersten Saugkörperlage 5 und eine maximale Quererstreckung der ersten Saugkörperlage 5 entspricht einer maximalen Quererstreckung des Absorptionskörpers 4. Alternativ können die erste Saugkörperlage 5 und der Absorptionskörper 4 in Querrichtung 10 oder Längsrichtung 11 des Absorptionskörpers unterschiedlich weit erstreckt sein.

Der Absorptionskörper 4 und ein gemeinsam durch das Topsheet 2 und das Backsheet 3 gebildetes Chassis 8 sind in diesem Ausführungsbeispiel anatomisch ausgebildet. Sie können jedoch auch rechteckig, dreieckig, oval, T-förmig, sanduhrförmig, asymmetrisch oder in einer anderen dem Fachmann als geeignet erscheinenden Form ausgebildet sein.

Der in **Figur 1a** dargestellte Inkontinenzartikel kann zusätzliche optionale Merkmale aufweisen, die im Stand der Technik hinreichend beschrieben sind, wie beispielsweise Elastifizierungsmittel, Flüssigkeitsbarrieren (z.B. Cuffs) oder Befestigungsmittel.

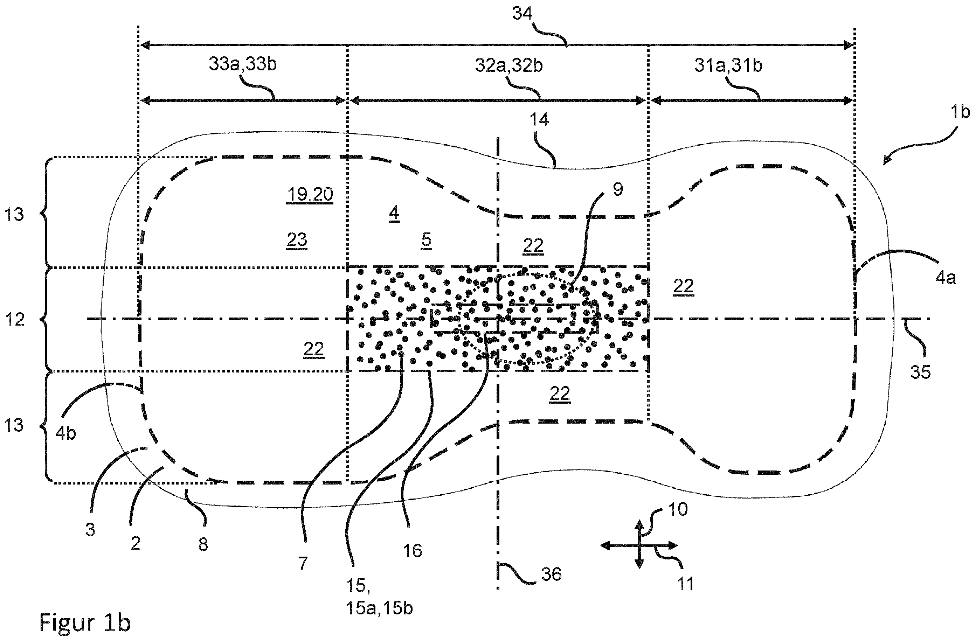

In **Figur 1b** ist ein Inkontinenzartikel 1b dargestellt, der die mit Bezug zu **Figur 1a** beschriebenen Merkmale aufweist sowie mit Bezug zu **Figur 1a** beschriebene optionale Merkmale aufweisen kann. Zusätzlich weist die erste Saugkörperlage 5 einen in der Längsrichtung 11 orientierten Kanal 16 auf.

Die Transferkomponente 15 überfängt in diesem Beispiel 18 % einer Gesamtfläche des Absorptionskörpers 4. Es wurde festgestellt, dass dieser Flächenanteil auf vorteilhafte Weise gleichzeitig eine effiziente Flüssigkeitsaufnahme und -Verteilung bietet und kostengünstig ist. Eine geeignete Transferkomponenet 15 kann jedoch zwischen 5 und 100 % der Gesamtfläche des Absorptionskörpers 4 überfangen.

In diesem Ausführungsbeispiel ist die Transferkomponente 15 symmetrisch zur Quermittelachse 36 des Absorptionskörpers 4 angeordnet. Die Transferkomponente 15 kann jedoch auch asymmetrisch zur Quermittelachse 36 des Absorptionskörpers 4 oder zu einer Quermittelachse des Inkontinenzartikels 1b angeordnet sein, mithin näher bei der vorderen Querkante 4a oder der hinteren Querkante 4b des Absorptionskörpers 4 als bei der jeweiligen anderen hinteren 4b oder vorderen Querkante 4a des Absorptionskörpers 4 angeordnet sein.

Sowohl die Transferkomponente 15 als auch der Kanal 16 erstrecken sich über den Miktionsbereich 9.

Der Kanal 16 ist parallel zu und auf der Längsmittelachse 35 des Absorptionskörpers 4 und des Inkontinenzartikels 1b angeordnet. Weiter ist der Kanal 16 symmetrisch zur Längsmittelachse 35 des Absorptionskörpers 4 und Inkontinenzartikels 1b ausgebildet.

Auf vorteilhafte Weise ist der Kanal 16 hier in Querrichtung 10 des Absorptionskörpers 4 zentriert angeordnet und erstreckt sich symmetrisch über die Quermittelachse 36 des Absorptionskörpers 4 hinweg.

Das Beispiel zeigt einen bevorzugten Kanal 16, der von der hinteren Querkante 4b des Absorptionskörpers 4 weiter entfernt ist als von der vorderen Querkante 4a des Absorptionskörpers 4. Jedoch kann der Kanal 16 auch gleich weit von der vorderen 4a und hinteren Querkante 4b des Absorptionskörpers 4 entfernt oder weiter von der vorderen 4a als von der hinteren Querkante 4b des Absorptionskörpers 4 entfernt angeordnet sein.

In diesem Beispiel ist der Kanal 16 rechteckig ausgebildet und erstreckt sich geradlinig in der Längsrichtung 11 des Absorptionskörpers 4 und Inkontinenzartikels 1b. Jedoch ist auch eine wellenförmige, gezackte oder bogenförmige oder jede andere Kanalform denkbar, die der Fachmann für sinnvoll erachtet. Unterschiedliche weitere Kanalformen wie beispielsweise mandelförmig, knochenförmig, T-förmig, dreieckig, oval, sternförmig oder verzweigt sind im Stand der Technik hinreichend bekannt.

Der Kanal 16 in diesem Beispiel weist auf vorteilhafte Weise eine größere Erstreckung in der Längsrichtung 11 des Absorptionskörpers 4 auf als in der Querrichtung 10.

Der Kanal 16 ist vollständig von der Transferkomponente 15 überfangen. Dabei kann eine im Benutzungszustand in den Kanal 16 geleitete Körperflüssigkeit zuvor auf das in der Transferkomponente 15 angeordnete pH-Regulationsmittel 7 treffen.

Ein einzelner zentraler Kanal 16 wie hier in der Figur 1b dargestellt wirkt sich vorteilhaft auf eine Stabilität und Form des Inkontinenzartikels 1b im Benutzungszustand aus. Jedoch ist auch denkbar, mehr als einen Kanal, insbesondere 2 Kanäle vorzusehen.

Die erste Saugkörperlage 5 weist einen ersten Bereich 22 auf, in welchem SAP 20 angeordnet ist. Der erste Bereich 22 der ersten Saugkörperlage 5 grenzt an den Kanal 16 an und umgibt den Kanal 16 vollständig.

Die **Figur 2a** zeigt in beispielhafter Weise schematisch, nicht maßstäblich, einen Querschnitt entlang der Quermittelachse eines Inkontinenzartikels 1c. Der Inkontinenzartikel 1c weist die mit Bezug zu **Figur 1a** beschriebenen Merkmale auf. Zusätzlich weist der Inkontinenzartikel 1c in dieser bevorzugten Ausführungsform eine Transferkomponente 15 auf.

Der Inkontinenzartikel 1c weist ein flüssigkeitsdurchlässiges Topsheet 2, ein im Wesentlichen (mithin in Gebrauch) flüssigkeitsundurchlässiges Backsheet 3, und einen dazwischen angeordneten Absorptionskörper 4 auf. Der Absorptionskörper 4 des Inkontinenzartikels 1c umfasst einen Speichersaugkörper 23 und eine Transferkomponente 15. Der Speichersaugkörper besteht in diesem Beispiel aus der ersten Saugkörperlage 5, die Fasermaterial 19 wie beispielsweise Zellstofffasern oder Kunststofffasern, und partikelförmiges SAP 20 aufweist. Die Transferkomponente umfasst eine obere Transferlage 15a und eine untere Transferlage 15b und pH-Regulationsmittel 7, das zwischen der oberen 15a und der unteren 15b Transferlage eingebracht ist. Das SAP 20 ist im gezeigten Fall gleichförmig verteilt zwischen der oberen 15a und der unteren Transferlage 15b angeordnet. Die Transferkomponente 15, mithin das pH-Regulationsmittel 7, ist zwischen der ersten Saugkörperlage 5 und dem Topsheet 2 angeordnet. Es ist vorteilhaft, wenn von der Topsheetseite eindringende Körperflüssigkeiten zunächst auf das pH-Regulierungsmittel 7 treffen bevor sie im Speichersaugkörper 23, insbesondere durch SAP 20, dauerhaft gebunden werden, da so eine rasche und effektivere pH-Wert-Kontrolle erfolgen kann.

Die obere Transferlage 15a und die untere Transferlage 15b sind in diesem Fall aus einem gleichen Vliesmaterial ausgebildet und weisen gleiche Eigenschaften auf.

Die obere 15a und die untere Transferlage 15b können sich auch in vorteilhafter Weise hinsichtlich mindestens einer Eigenschaft ausgewählt aus der Gruppe Flächengewicht, Dichte, Reißfestigkeit, Opazität, Rücknässung, Flüssigkeitsdurchtrittszeit (Strike Through Time), Erstreckung in Längs- oder Quer- oder Dickenrichtung, Materialart, Faserart, Faserlänge, Faserdurchmesser, Faserkräuselung, Hydrophobizität, Art oder Menge an Additiven, voneinander unterscheiden.

Das pH-Regulationsmittel 7 kann auf vorteilhafte Weise zumindest teilweise und wenigstens an einer der oberen 15a oder unteren Transferlage 15b immobilisiert, insbesondere mittels eines Klebstoffs immobilisiert sein (in **Figur 2a** nicht dargestellt).

Weiterhin kann der Inkontinenzartikel 1c eine oder mehrere zusätzliche absorbierende Schichten oder Transferlagen umfassen.

Die **Figur 2b** zeigt schematisch, nicht maßstäblich, einen Querschnitt entlang der Quermittelachse eines weiteren bevorzugten Inkontinenzartikels 1d. Der Inkontinenzartikel 1c weist die mit Bezug zu **Figur 2a** beschriebenen Merkmale auf und kann zusätzlich die mit Bezug zu **Figur 2a** beschriebenen optionalen Merkmale aufweisen. Zusätzlich weist der Speichersaugkörper 23 des Inkontinenzartikels 1c in dieser bevorzugten Ausführungsform neben der ersten Saugkörperlage 5 noch eine zweite Saugkörperlage 6 auf.

Die zweite Saugkörperlage 6 ist unterhalb der ersten Saugkörperlage 5 angeordnet, also zwischen erster Saugkörperlage 5 und dem Backsheet 3. In dieser bevorzugten Ausführungsform weist die zweite Saugkörperlage 6 kein SAP auf. Weiterhin weist die zweite Saugkörperlage 6 auch kein pH-Regulierungsmittel 7 auf. Dadurch wird der Tragekomfort des Inkontinenzartikels von der Backsheetseite verbessert und die Gefahr von Beschädigungen des Backsheets durch Partikel verringert. Alternativ können geringe Mengen an SAP und/oder pH-Regulierungsmittel in der zweiten Saugkörperlage 6 vorgesehen sein.

Die zweite Saugkörperlage 6 besteht im Wesentlichen aus Fasermaterial 19, wie beispielsweise Zellstofffasern oder Kunststofffasern.

Die **Figur 2c** zeigt schematisch, nicht maßstäblich, einen Querschnitt entlang der Quermittelachse eines weiteren bevorzugten Inkontinenzartikels 1e. Der Inkontinenzartikel 1e weist mit Bezug zu **Figur 2a** beschriebenen Merkmale auf. Zusätzlich sind die obere 15a und die untere Transferlage 15b durch eine Falzkante 24 miteinander verbunden. Die Falzkante 24 verläuft in der hier nicht dargestellten Längsrichtung des Inkontinenzartikels 1e. Die Transferkomponente 15 ist in diesem bevorzugten Beispiel einstückig ausgebildet. Alternativ können die obere 15a und untere Transferlage 15b auch durch eine, insbesondere in einer Längsrichtung verlaufende Fügestelle miteinander verbunden sein. Das Fügen der oberen und unteren Transferlage kann hierbei mittels jedem dem Fachmann an sich bekannten und zu dem Zweck und entsprechend dem Material der oberen und unteren Transferlage geeigneten Fügeverfahren erfolgen, beispielsweise Thermoschweißen, Nähen, Siegeln, Kleben oder Ultraschallschweißen.

Die **Figur 2d** zeigt schematisch, nicht maßstäblich, einen Querschnitt entlang der Quermittelachse eines weiteren bevorzugten Inkontinenzartikels 1f. Der Inkontinenzartikel 1f weist die mit Bezug zu **Figur 2b** beschriebenen Merkmale auf. Zusätzlich sind die obere 15a und die untere Transferlage 15b durch eine Falzkante 24 miteinander verbunden. Die Falzkante 24 verläuft in der hier nicht dargestellten Längsrichtung des Inkontinenzartikels 1f. Die Transferkomponente 15 ist in diesem bevorzugten Beispiel einstückig ausgebildet. Alternativ können die obere 15a und die untere Transferlage 15b auch mittels einer wie mit Bezug zu **Figur 2c** beschriebenen Fügestelle miteinander gefügt sein.

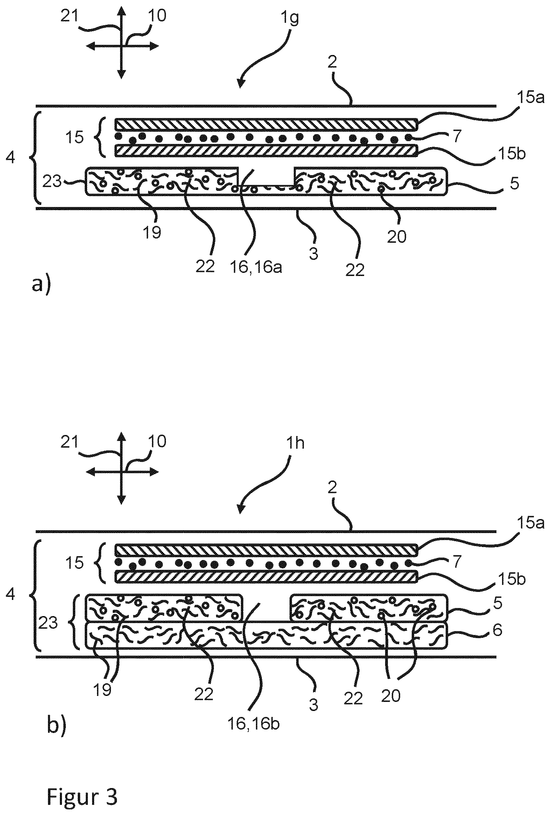

Die **Figur 3a** zeigt schematisch, nicht maßstäblich, einen Querschnitt entlang der Quermittelachse eines weiteren bevorzugten Inkontinenzartikels 1g. Der Inkontinenzartikel 1g weist die mit Bezug zu **Figur 2a** beschriebenen Merkmale auf. Zusätzlich weist der Inkontinenzartikel 1g in dieser bevorzugten Ausführungsform noch einen Kanal 16 auf.

Der Kanal 16,16a erstreckt sich in diesem Beispiel nicht vollständig durch die erste Saugkörperlage 5 hindurch. Dabei kann der Kanal 16 beispielsweise mittels Prägen oder Komprimieren der ersten Saugkörperlage 5 ausgebildet sein.

Dabei ist der Kanal 16,16a im Wesentlichen frei von absorbierenden Materialien, nämlich SAP 20 und Fasermaterial 19. Der Kanal 16 kann alternativ, wie nachfolgend in Bezug zu **Figur 3b** näher beschrieben, als eine sich durch die erste Saugkörperlage 5 in Dickenrichtung 21 hindurcherstreckende Ausnehmung gebildet sein.

In dieser bevorzugten Ausführungsform weist die erste Saugkörperlage 5 einen einzigen Kanal 16 auf, der auf einer hier nicht dargestellten Längsmittelachse des Absorptionskörpers 4 angeordnet ist. Weiter ist der Kanal 16 symmetrisch zur Längsmittelachse des Absorptionskörpers 4 ausgebildet. Der Kanal 16 ist auf vorteilhafte Weise in Querrichtung 10 des Absorptionskörpers 4 zentriert angeordnet.

Alternativ kann die erste Saugkörperlage mehr als einen Kanal, insbesondere 2-5, weiter insbesondere genau 2 Kanäle aufweisen.

Die erste Saugkörperlage 5 weist einen ersten Bereich 22 auf, der an den Kanal 16 angrenzt und der die gesamte Menge des SAP 20 der ersten Saugkörperlage 5 enthält.

Die **Figur 3b** zeigt schematisch, nicht maßstäblich, einen Querschnitt entlang der Quermittelachse eines weiteren bevorzugten Inkontinenzartikels 1h.

Der Inkontinenzartikel 1h weist die mit Bezug zu **Figur 3a** beschriebenen Merkmale auf. Zusätzlich weist der Speichersaugkörper 23 des Inkontinenzartikels 1h in dieser bevorzugten Ausführungsform neben der ersten Saugkörperlage 5 noch eine zweite Saugkörperlage 6 auf. Die Merkmale der zweiten Saugkörperlage 6 sind mit Bezug zu **Figur 2b** näher beschrieben. Der Kanal 16 ist in diesem bevorzugten Ausführungsbeispiel als eine sich durch die erste Saugkörperlage 5 in Dickenrichtung 21 hindurcherstreckende Ausnehmung 16b gebildet. Der Kanal 16 kann ebenso wie mit Bezug zu **Figur 3a** näher beschrieben als eine sich in der ersten Saugkörperlage 5 in Dickenrichtung 21 erstreckende Ausnehmung 16a gebildet sein. Als eine alternative Ausführungsform kann der Kanal 16 auch mittels Prägen oder Komprimieren der ersten Saugkörperlage 5 ausgebildet sein.

Der Kanal 16,16b der ersten Saugkörperlage 5 ist in diesem bevorzugten Beispiel im Wesentlichen frei von absorbierenden Materialien, nämlich SAP 20 und Fasermaterial 19.

In den in **Figuren 1a****,****b****,** **2a-d** **und** **3a,b** dargestellten Ausführungsbeispielen umfasst das pH-Regulationsmittel 7 partikelförmiges Mononatriumcitrat, Typ "Fine Granular F3500" von Jungbunzlauer. Dieses Mononatriumcitrat weist zu 59 Gewichtsprozent eine Partikelgröße von 80 bis 355 µm und eine Reinheit von mindestens 99 % auf.

In den in **Figuren 1a****,****b****,** **2a-d** **und** **3a,b** dargestellten Ausführungsbeispielen ist das SAP 20 partikelförmig ausgebildet. In einer denkbaren Variante kann das SAP faserförmig oder blatt- oder schaumförmig ausgebildet sein.

Die Transferkomponente 15 überfängt die erste Saugkörperlage 5 des Absorptionskörpers 4 nur teilweise und weist vorliegend eine geringere Erstreckung als die erste Saugkörperlage 5 **(****Figuren 2a****-d und 3a,b**) und gegebenenfalls die zweite Saugkörperlage 6 (**Figuren 2b****,d und 3b**) auf. Alternativ kann die die Transferkomponente 15 die erste Saugkörperlage 5 und/oder die zweite Saugkörperlage 6 vollständig überfangen.

Die erste Saugkörperlage 5 ist in den dargestellten bevorzugten Beispielen (**Figuren 2b****,d und 3b**) in der Querrichtung 10 im Wesentlichen gleich erstreckt wie die zweite Saugkörperlage 6 des Speichersaugkörpers 23. Die erste Saugkörperlage 5 und die zweite Saugkörperlage 6 können aber in der Querrichtung 10 auch unterschiedlich erstreckt sein. Der erste Bereich 22 der ersten Saugkörperlage 5 und die zweite Saugkörperlage 6 sind in den beschriebenen bevorzugten Ausführungsbeispielen als gleichförmig dicke Lagen, also mit einer gleichförmigen Erstreckung in der Dickenrichtung 21, dargestellt. Gleichwohl kann der erste Bereich 22 der ersten Saugkörperlage 5 und/oder die zweite Saugkörperlage 6 ein Dickenprofil aufweisen.

Die in **Figuren 2a****-d, 3a und 3b** dargestellten Ausführungsformen sind so zu verstehen, dass sie ein oder mehrere weitere typische Merkmale von Inkontinenzartikeln aufweisen können, insbesondere dass sie als verschiedene Typen von Inkontinenzartikeln ausgebildet sein können wie beispielsweise Inkontinenzvorlagen, Inkontinenzeinlagen, Slipeinlagen, Windeln des offenen oder des geschlossenen Typs oder Krankenunterlagen.

### Testmethode pH-Wert-Messung an der Oberfläche eines Inkontinenzartikels:

Zur Durchführung einer pH-Wert-Messung an der Oberfläche eines Inkontinenzartikels, insbesondere auf einer äußeren Oberseite des Topsheets des Inkontinenzartikels, wird eine Urinersatzlösung verwendet. Die Urinersatzlösung ist eine nach ISO 11984-1 (1996) hergestellte 0,9 %ige (w/v) Lösung aus Natriumchlorid (NaCl) in destilliertem Wasser, welche mit Natriumhydroxid (NaOH) auf pH 6.8 eingestellt ist. Eine solche Lösung ist im allgemeinen Fachwissen seit langem bekannt und deren Herstellung gehört zu den Routinemethoden des Fachgebiets.

Der zu testende Inkontinenzartikel wird mit der Topsheet-Seite nach oben flach ausgebreitet, dabei gegebenenfalls vollständig aufgefaltet und gegebenenfalls auf einer Unterlage fixiert. Der Test wird entsprechend der ISO 11984-1 (1996) bei einer Temperatur von 23 °C ± 2 °C und einer relativen Luftfeuchtigkeit von 50% ± 5% durchgeführt. Der zu testende Inkontinenzartikel und die Urinersatzflüssigkeit werden gemäß ISO 11984-1 (1996) entsprechend vorkonditioniert.

Um eine Benutzungssituation relativ realitätsnah abzubilden und die pH-Wert-Entwicklung oder die Pufferwirkung des pH-Regulationsmittels über eine längere Benutzungsdauer mit mehreren Miktionsereignissen zu verfolgen, werden wie nachfolgend beschrieben im Verlauf des Tests drei Miktionsereignisse über einen Zeitraum von fünf Stunden simuliert. Der Inkontinenzartikel wird hierbei insgesamt mit einem Flüssigkeitsvolumen beladen, das etwa 90% einer maximalen Absorptionskapazität des Inkontinenzartikels entspricht.

Die Volumina der ersten und zweiten und dritten simulierten Miktion sind daher so zu wählen, dass ein Gesamtvolumen der ersten und zweiten und dritten simulierten Miktion etwa 90% der maximalen Absorptionskapazität des Inkontinenzartikels entspricht. Dabei entspricht ein Volumen einer ersten simulierten Miktion 45% bis 50% der maximalen Absorptionskapazität des Inkontinenzartikels. Ein jeweiliges Volumen einer zweiten und einer dritten simulierten Miktion ist im Wesentlichen gleich und entspricht jeweils 20% bis 22,5% der maximalen Absorptionskapazität.

Falls die maximale Absorptionskapazität eines Inkontinenzartikels nicht bekannt ist, wird die maximale Absorptionskapazität anhand eines zweiten vergleichbaren Inkontinenzartikels, beispielsweise eines derselben Saugstärke und Größe und Produktart zuzuordnenden und gegebenenfalls derselben Packung zu entnehmenden Inkontinenzartikels wie der zu testende Inkontinenzartikel, vorab gemäß ISO 11984-1 (1996) bestimmt.

Ein Volumen der Urinersatzlösung, welches 50% der maximalen Absorptionskapazität des Inkontinenzartikels entspricht, wird innerhalb von 30 Sekunden zum Zweck der Simulierung einer ersten Miktion auf den trockenen Inkontinenzartikel gegossen und zwar derart, dass die Urinersatzlösung in einem Bereich auf den Inkontinenzartikel trifft, in welchem auch bei einem Miktionsereignis im Benutzungszustand die Miktionsflüssigkeit auf den Inkontinenzartikel treffen würde. Sollte dieser Bereich nicht zweifelsfrei feststehen, soll die Urinersatzlösung mittig auf den Absorptionskörper, insbesondere im Bereich einer Quermittelachse des Absorptionskörpers treffen. Falls der Inkontinenzartikel eine Transferkomponente aufweist, wird die Urinersatzlösung insbesondere mittig innerhalb eines von der Transferkomponente gebildeten Bereiches auf den Inkontinenzartikel gegossen. Zwei Stunden nach Beginn der ersten simulierten Miktion mit dem ersten Volumen der Urinersatzlösung wird ein zweites Volumen der Urinersatzlösung, welches 20 % der maximalen Absorptionskapazität des Inkontinenzartikels entspricht, wie oben beschrieben auf den Inkontinenzartikel gegossen, um eine zweite Miktion zu simulieren.

Fünf Stunden nach Beginn der Beladung mit dem ersten Volumen der Urinersatzlösung wird ein drittes Volumen der Urinersatzlösung, welches 20 % der maximalen Absorptionskapazität des Inkontinenzartikels entspricht, wie oben beschrieben auf den Inkontinenzartikel gegossen, um eine dritte Miktion zu simulieren.

Nach jedem der drei simulierten Miktionsereignisse wird der pH-Wert an der äußeren Oberseite des Topsheets des Inkontinenzartikels 1, 3, 5, 10, 15 und 20 min nach Ende der jeweiligen simulierten Miktion gemessen. Dazu wird der pH-Wert an jeweils 4 Messpunkten gemessen, welche in Längs- und/oder Querrichtung des flach ausgebreiteten Inkontinenzartikels voneinander beabstandet sind und ein Durchschnittswert der vier gemessenen Werte gebildet. Falls eine Transferkomponente vorhanden ist, sollen die Messungen innerhalb des von der Transferkomponente gebildeten Bereiches erfolgen. In jedem Fall sind die Messpunkte innerhalb eines pH-Regulationsmittel aufweisenden Bereiches anzuordnen.

Die Messpunkte sollen voneinander beabstandet sein, insbesondere zumindest nicht miteinander überlappen. Es ist grundsätzlich darauf zu achten, dass die Messpunkte des Inkontinenzartikels nicht zu trocken sind, um verlässliche Messergebnisse zu erhalten. Bevorzugt sollen die Positionen der 4 Messpunkte bei allen Messungen beibehalten werden, jedoch ist auch möglich, die Position eines oder mehrerer Messpunkte zu variieren, beispielsweise falls ein Messpunkt zum Zeitpunkt einer späteren Messung zu trocken sein sollte. Das kann beispielsweise bei Inkontinenzartikeln mit besonders niedriger Rücknässung insbesondere 15 und/oder 20 Minuten nach der jeweiligen simulierten Miktion der Fall sein. Sollten nicht wenigstens zwei geeignete Messpunkte für eine Messung zur Verfügung stehen, wird die Messung nur zu den Zeitpunkten 1, 3, 5, 10 und gegebenenfalls 15 Minuten nach Ende der jeweiligen simulierten Miktion ausgewertet. Sollten für eine Messung nur zwei oder drei Messpunkte zur Verfügung stehen ist ein Durchschnittswert der zwei oder drei gemessenen Werte zu bilden.

Aufgrund des Routine-Charakters solcher pH-Messungen im Fachgebiet ist es dem Fachmann leicht möglich, eine Beurteilung vorzunehmen, ob ein möglicher Messpunkt zu trocken ist.

Die Messung kann grundsätzlich mit jedem Messgerät vorgenommen werden, das sich nach Einschätzung des Fachmanns für die Oberflächenmessung von pH-Werten eignet. Beispielsweise eignen sich die Elektroden der Modelle HI14142 oder HI1413 von HANNA Instruments. Das Messgerät wird vor Beginn der Messung entsprechend der Herstellerangaben kalibriert.

## Patentansprüche

1. Inkontinenzartikel für die Aufnahme von Körperausscheidungen, umfassend ein zumindest bereichsweise flüssigkeitsdurchlässiges Topsheet (2), ein im Wesentlichen flüssigkeitsundurchlässiges Backsheet (3) und einen zwischen dem Topsheet (2) und dem Backsheet (3) angeordneten Absorptionskörper (4) wobei der Absorptionskörper (4) einen Speichersaugkörper (23) und eine Transferkomponente (15) aufweist, wobei die Transferkomponente (15) zwischen dem Speichersaugkörper (23) und dem Topsheet (2) angeordnet ist, und wobei die Transferkomponente (15) zumindest eine obere Transferlage (15a) und eine untere Transferlage (15b) aufweist, und wobei ein pH-Regulationsmittel (7) zwischen der oberen Transferlage (15a) und der unteren Transferlage (15b) eingebracht ist.

2. Inkontinenzartikel nach Anspruch 1 **dadurch gekennzeichnet, dass** die obere Transferlage (15a) sich hinsichtlich mindestens einer Eigenschaft ausgewählt aus der Gruppe Flächengewicht, Dichte, Reißfestigkeit, Opazität, Rücknässung, Flüssigkeitsdurchtrittszeit (Strike Through Time), Erstreckung in Längs- oder Quer- oder Dickenrichtung, Materialart, Faserart, Faserlänge, Faserdurchmesser, Faserkräuselung, Hydrophobizität, Art oder Menge an Additiven, von der unteren Transferlage (15b) unterscheidet.

3. Inkontinenzartikel nach Anspruch 1 oder 2 **dadurch gekennzeichnet, dass** die obere Transferlage (15a) und die untere Transferlage (15b) durch eine, insbesondere in einer Längsrichtung (11) verlaufende Falzkante (24) miteinander verbunden sind.

4. Inkontinenzartikel nach einem oder mehreren der vorhergehenden Ansprüche **dadurch gekennzeichnet, dass** das pH-Regulationsmittel (7) gleichförmig verteilt zwischen der oberen (15a) und der unteren Transferlage (15b) eingebracht ist.

5. Inkontinenzartikel nach einem oder mehreren der vorhergehenden Ansprüche **dadurch gekennzeichnet, dass** der Speichersaugkörper (23) eine erste Saugkörperlage (5) aufweist, wobei die erste Saugkörperlage (5) superabsorbierendes Polymer (SAP) (20) aufweist.

6. Inkontinenzartikel nach Anspruch 5 **dadurch gekennzeichnet, dass** das SAP (20) in der ersten Saugkörperlage (5) gleichförmig verteilt eingebracht ist insbesondere derart, dass die Konzentration des SAP (20) in Gewichtsprozent in einem mittleren Längsabschnitt (32a) der ersten Saugkörperlage (5) gleich groß ist wie in einem vorderen (31a) und/oder einem hinteren Längsabschnitt (33a) der ersten Saugkörperlage (5).

7. Inkontinenzartikel nach einem oder mehreren der vorhergehenden Ansprüche **dadurch gekennzeichnet, dass** die flächenbezogene Menge des pH-Regulationsmittels (7) in einem mittleren Längsabschnitt (32b) des Absorptionskörpers (4) in g/m² größer ist als in einem vorderen (31b) und/oder einem hinteren Längsabschnitt (33b) des Absorptionskörpers (4).

8. Inkontinenzartikel nach einem oder mehreren der vorhergehenden Ansprüche **dadurch gekennzeichnet, dass** die erste Saugkörperlage (5) einen in der Längsrichtung (11) orientierten Kanal (16) aufweist.

9. Inkontinenzartikel nach Anspruch 8 **dadurch gekennzeichnet, dass** die Transferkomponente (15) den Kanal (16) zumindest bereichsweise, insbesondere vollständig überfängt.

10. Inkontinenzartikel nach einem oder mehreren der Ansprüche 5 bis 9 **dadurch gekennzeichnet, dass** die erste Saugkörperlage (5) einen ersten Bereich (22) aufweist, und wobei die erste Saugkörperlage (5) in dem ersten Bereich (22) SAP (20) aufweist, wobei insbesondere der erste Bereich (22) an den Kanal (16) angrenzt, weiter insbesondere dass der erste Bereich (22) den Kanal (16) vollständig umgibt.

11. Inkontinenzartikel nach einem oder mehreren der Ansprüche 5 bis 10 **dadurch gekennzeichnet, dass** der Speichersaugkörper (23) eine zweite Saugkörperlage (6) aufweist, wobei die zweite Saugkörperlage (6) unterhalb der ersten Saugkörperlage (5), also zwischen der ersten Saugkörperlage (5) und dem Backsheet (3) angeordnet ist.

12. Inkontinenzartikel nach Anspruch 11 **dadurch gekennzeichnet, dass** die zweite Saugkörperlage (6) weniger als 20 Gewichtsprozent, insbesondere weniger als 15 Gewichtsprozent, weiter insbesondere weniger als 10 Gewichtsprozent, weiter insbesondere weniger als 5 Gewichtsprozent SAP (20) aufweist, insbesondere frei von SAP (20) ist.

13. Inkontinenzartikel nach einem oder mehreren der Ansprüche 8 bis 12 **dadurch gekennzeichnet, dass** der Kanal (16) eine sich durch die erste Saugkörperlage (5) in Dickenrichtung (21) hindurcherstreckende Ausnehmung umfasst oder daraus gebildet ist.

14. Inkontinenzartikel nach einem oder mehreren der Ansprüche 8 bis 13 **dadurch gekennzeichnet, dass** der Kanal (16) auf einer Längsmittelachse (35) des Inkontinenzartikels angeordnet ist.

15. Inkontinenzartikel nach einem oder mehreren der vorhergehenden Ansprüche **dadurch gekennzeichnet, dass** das pH-Regulationsmittel (7) partikelförmig ausgebildet ist, wobei insbesondere mindestens 50 Gewichtsprozent des pH-Regulationsmittels (7) eine Partikelgröße von 10 bis 2000 µm, weiter insbesondere von 50 bis 1200 µm, weiter insbesondere von 80 bis 800 µm aufweist.

16. Inkontinenzartikel nach einem oder mehreren der vorhergehenden Ansprüche **dadurch gekennzeichnet, dass** die flächenbezogene Menge an pH-Regulationsmittel (7) in der Transferkomponente (15) 5 bis 100 g/m², insbesondere 7 bis 90 g/m², weiter insbesondere 10 bis 80 g/m² beträgt.

17. Inkontinenzartikel nach einem oder mehreren der vorhergehenden Ansprüche **dadurch gekennzeichnet, dass** das pH-Regulationsmittel (7) Trinatriumcitrat, Mononatriumcitrat oder Dinatriumcitrat aufweist, insbesondere dass das pH-Regulationsmittel (7) aus Mononatriumcitrat oder Dinatriumcitrat besteht.

18. Inkontinenzartikel nach einem oder mehreren der Ansprüche 8 bis 17 **dadurch gekennzeichnet, dass** der Kanal (16) der ersten Saugkörperlage (5) im Wesentlichen frei von SAP (20) ist.

19. Inkontinenzartikel nach einem oder mehreren der Ansprüche 11 bis 18 **dadurch gekennzeichnet, dass** die erste (5) und/oder die zweite Saugkörperlage (6) weniger als 20 Gewichtsprozent, insbesondere weniger als 15 Gewichtsprozent, weiter insbesondere weniger als 10 Gewichtsprozent, weiter insbesondere weniger als 5 Gewichtsprozent des pH-Regulationsmittels (7) aufweist, insbesondere frei von pH-Regulationsmittel (7) ist.

20. Inkontinenzartikel nach einem oder mehreren der vorhergehenden Ansprüche **dadurch gekennzeichnet, dass** das pH-Regulationsmittel (7) zumindest teilweise und wenigstens an einer der oberen (15a) oder unteren Transferlage (15b) immobilisiert, insbesondere mittels eines Klebstoffs immobilisiert ist.

## Claims

1. Incontinence article for absorption of bodily excretions, comprising an at least regionally liquid-permeable topsheet (2), a substantially liquid-impermeable backsheet (3), and an absorption body (4) arranged between the topsheet (2) and the backsheet (3), wherein the absorption body (4) comprises a storage absorbent (23) and a transfer component (15), wherein the transfer component (15) is arranged between the storage absorbent (23) and the topsheet (2), and wherein the transfer component (15) comprises at least an upper transfer ply (15a) and a lower transfer ply (15b), and wherein a pH regulator (7) is introduced between the upper transfer ply (15a) and the lower transfer ply (15b).

2. Incontinence article according to Claim 1, **characterized in that** the upper transfer ply (15a) differs from the lower transfer ply (15b) with respect to at least one property selected from the group consisting of basis weight, density, tear strength, opacity, rewetting, liquid passage time (strike through time), extent in longitudinal or transverse or thickness direction, material type, fiber type, fiber length, fiber diameter, fiber crimp, hydrophobicity, and type or amount of additives.

3. Incontinence article according to Claim 1 or 2, **characterized in that** the upper transfer ply (15a) and the lower transfer ply (15b) are connected to one another by a folded edge (24) running in particular in a longitudinal direction (11).

4. Incontinence article according to one or more of the preceding claims, **characterized in that** the pH regulator (7) is introduced between the upper transfer ply (15a) and the lower transfer ply (15b) with a uniform distribution.

5. Incontinence article according to one or more of the preceding claims, **characterized in that** the storage absorbent (23) comprises a first absorbent ply (5), wherein the first absorbent ply (5) comprises superabsorbent polymer (SAP) (20).

6. Incontinence article according to Claim 5, **characterized in that** the SAP (20) is introduced in the first absorbent ply (5) with a uniform distribution, in particular in such a way that the concentration of the SAP (20) in percent by weight is the same in a middle longitudinal section (32a) of the first absorbent ply (5) as in a front longitudinal section (31a) and/or a rear longitudinal section (33a) of the first absorbent ply (5).

7. Incontinence article according to one or more of the preceding claims, **characterized in that** the amount per unit area of the pH regulator (7) is, in g/m², greater in a middle longitudinal section (32b) of the absorption body (4) than in a front longitudinal section (31b) and/or a rear longitudinal section (33b) of the absorption body (4).

8. Incontinence article according to one or more of the preceding claims, **characterized in that** the first absorbent ply (5) comprises a channel (16) oriented in the longitudinal direction (11).

9. Incontinence article according to Claim 8, **characterized in that** the transfer component (15) covers the channel (16) at least regionally, in particular completely.

10. Incontinence article according to one or more of Claims 5 to 9, **characterized in that** the first absorbent ply (5) has a first region (22), and wherein the first absorbent ply (5) comprises SAP (20) in the first region (22), wherein, in particular, the first region (22) adjoins the channel (16), and further in particular **in that** the first region (22) completely surrounds the channel (16).

11. Incontinence article according to one or more of Claims 5 to 10, **characterized in that** the storage absorbent (23) comprises a second absorbent ply (6), wherein the second absorbent ply (6) is arranged below the first absorbent ply (5), i.e., between the first absorbent ply (5) and the backsheet (3).

12. Incontinence article according to Claim 11, **characterized in that** the second absorbent ply (6) comprises less than 20 percent by weight, in particular less than 15 percent by weight, further in particular less than 10 percent by weight, further in particular less than 5 percent by weight of SAP (20), and in particular is free of SAP (20).

13. Incontinence article according to one or more of Claims 8 to 12, **characterized in that** the channel (16) comprises or is formed from a cutout which extends through the first absorbent ply (5) in the thickness direction (21).

14. Incontinence article according to one or more of Claims 8 to 13, **characterized in that** the channel (16) is arranged on a longitudinal central axis (35) of the incontinence article.

15. Incontinence article according to one or more of the preceding claims, **characterized in that** the pH regulator (7) is particulate, wherein, in particular, at least 50 percent by weight of the pH regulator (7) has a particle size of 10 to 2000 µm, further in particular of 50 to 1200 µm, further in particular of 80 to 800 µm.

16. Incontinence article according to one or more of the preceding claims, **characterized in that** the amount per unit area of pH regulator (7) in the transfer component (15) is 5 to 100 g/m², in particular 7 to 90 g/m², further in particular 10 to 80 g/m².

17. Incontinence article according to one or more of the preceding claims, **characterized in that** the pH regulator (7) comprises trisodium citrate, monosodium citrate or disodium citrate, in particular **in that** the pH regulator (7) consists of monosodium citrate or disodium citrate.

18. Incontinence article according to one or more of Claims 8 to 17, **characterized in that** the channel (16) of the first absorbent ply (5) is substantially free of SAP (20).

19. Incontinence article according to one or more of Claims 11 to 18, **characterized in that** the first absorbent ply (5) and/or the second absorbent ply (6) comprises less than 20 percent by weight, in particular less than 15 percent by weight, further in particular less than 10 percent by weight, further in particular less than 5 percent by weight of the pH regulator (7), and in particular is free of pH regulator (7).

20. Incontinence article according to one or more of the preceding claims, **characterized in that** the pH regulator (7) is immobilized at least partially and at least on a transfer ply of the upper transfer ply (15a) or lower transfer ply (15b), in particular immobilized by means of an adhesive.

## Revendications

1. Article pour incontinence destiné à recueillir des excrétions corporelles, comprenant une feuille supérieure (2) perméable aux liquides au moins par zones, une feuille arrière (3) sensiblement imperméable aux liquides et un corps absorbant (4) agencé entre la feuille supérieure (2) et la feuille arrière (3), le corps absorbant (4) présentant un absorbant d'accumulation (23) et un composant de transfert (15), le composant de transfert (15) étant agencé entre l'absorbant d'accumulation (23) et la feuille supérieure (2) et le composant de transfert (15) présentant au moins une couche de transfert supérieure (15a) et une couche de transfert inférieure (15b) et un régulateur de pH (7) étant incorporé entre la couche de transfert supérieure (15a) et la couche de transfert inférieure (15b).

2. Article pour incontinence selon la revendication 1, **caractérisé en ce que** la couche de transfert supérieure (15a) se distingue en ce qui concerne au moins une propriété, choisie dans le groupe formé par le poids surfacique, la densité, la résistance à la déchirure, l'opacité, le mouillage en retour, le temps de passage de liquide (Strike Through Time), l'extension dans la direction longitudinale ou transversale ou de l'épaisseur, le type de matériau, le type de fibres, la longueur des fibres, le diamètre des fibres, l'ondulation des fibres, l'hydrophobicité, le type ou la quantité d'additifs, de celle de la couche de transfert inférieure (15b).

3. Article pour incontinence selon la revendication 1 ou 2, **caractérisé en ce que** la couche de transfert supérieure (15a) et la couche de transfert inférieure (15b) sont reliées l'une à l'autre par une arête de pliage (24) s'étendant en particulier dans une direction longitudinale (11).

4. Article pour incontinence selon l'une ou plusieurs des revendications précédentes, **caractérisé en ce que** le régulateur de pH (7) est incorporé de manière régulièrement répartie entre la couche de transfert supérieure (15a) et la couche de transfert inférieure (15b).

5. Article pour incontinence selon l'une ou plusieurs des revendications précédentes, **caractérisé en ce que** l'absorbant d'accumulation (23) présente une première couche absorbante (5), la première couche absorbante (5) présentant un polymère superabsorbant (SAP) (20).

6. Article pour incontinence selon la revendication 5, **caractérisé en ce que** le SAP (20) est incorporé de manière régulièrement répartie dans la première couche absorbante (5), en particulier de telle sorte que la concentration en SAP (20), en pourcentage en poids, dans une section longitudinale centrale (32a) de la première couche absorbante (5) est égale à celle dans une section longitudinale avant (31a) et/ou arrière (33a) de la première couche absorbante (5).

7. Article pour incontinence selon l'une ou plusieurs des revendications précédente, **caractérisé en ce que** la quantité, rapportée à la surface, du régulateur de pH (7) dans une section longitudinale centrale (32b) du corps absorbant (4), en g/m², est supérieure à celle dans une section longitudinale avant (31b) et/ou arrière (33b) du corps absorbant (4).

8. Article pour incontinence selon l'une ou plusieurs des revendications précédentes, **caractérisé en ce que** la première couche absorbante (5) présente un canal (16) orienté dans la direction longitudinale (11).

9. Article pour incontinence selon la revendication 8, **caractérisé en ce que** le composant de transfert (15) recouvre le canal (16) au moins partiellement, en particulier complètement.

10. Article pour incontinence selon l'une ou plusieurs des revendications 5 à 9, **caractérisé en ce que** la première couche absorbante (5) présente une première zone (22) et la première couche absorbante (5) présentant un SAP (20) dans la première zone (22), la première zone (22) étant en particulier adjacente au canal (16), plus particulièrement, la première zone (22) entourant complètement le canal (16).

11. Article pour incontinence selon l'une ou plusieurs des revendications 5 à 10, **caractérisé en ce que** l'absorbant d'accumulation (23) présente une deuxième couche absorbante (6), la deuxième couche absorbante (6) étant agencée sous la première couche absorbante (5), c'est-à-dire entre la première couche absorbante (5) et la feuille arrière (3).

12. Article pour incontinence selon la revendication 11, **caractérisé en ce que** la deuxième couche absorbante (6) présente moins de 20% en poids, en particulier moins de 15% en poids, plus particulièrement moins de 10% en poids, plus particulièrement moins de 5% en poids, de SAP (20) et est en particulier exempte de SAP (20).

13. Article pour incontinence selon l'une ou plusieurs des revendications 8 à 12, **caractérisé en ce que** le canal (16) comprend ou est formé par un évidement s'étendant à travers la première couche absorbante (5) dans la direction de l'épaisseur (21).

14. Article pour incontinence selon l'une ou plusieurs des revendications 8 à 13, **caractérisé en ce que** le canal (16) est agencé sur un axe central longitudinal (35) de l'article pour incontinence.

15. Article pour incontinence selon l'une ou plusieurs des revendications précédentes, **caractérisé en ce que** le régulateur de pH (7) est réalisé sous forme de particules, en particulier au moins 50% en poids du régulateur de pH (7) présentant une grosseur de particule de 10 à 2000 µm, plus particulièrement de 50 à 1200 µm, plus particulièrement de 80 à 800 µm.

16. Article pour incontinence selon l'une ou plusieurs des revendications précédentes, **caractérisé en ce que** la quantité, rapportée à la surface, de régulateur de pH (7) dans le composant de transfert (15) est de 5 à 100 g/m², en particulier de 7 à 90 g/m², plus particulièrement de 10 à 80 g/m².

17. Article pour incontinence selon l'une ou plusieurs des revendications précédentes, **caractérisé en ce que** le régulateur de pH (7) présente du citrate trisodique, du citrate monosodique ou du citrate disodique, en particulier **en ce que** le régulateur de pH (7) est constitué de citrate monosodique ou de citrate disodique.

18. Article pour incontinence selon l'une ou plusieurs des revendications 8 à 17, **caractérisé en ce que** le canal (16) de la première couche absorbante (5) est sensiblement exempt de SAP (20).

19. Article pour incontinence selon l'une ou plusieurs des revendications 11 à 18, **caractérisé en ce que** la première (5) et/ou la deuxième (6) couche absorbante présente(nt) moins de 20% en poids, en particulier moins de 15% en poids, plus particulièrement moins de 10% en poids, plus particulièrement moins de 5% en poids, du régulateur de pH (7) et est en particulier exempte de régulateur de pH (7).

20. Article pour incontinence selon l'une ou plusieurs des revendications précédentes, **caractérisé en ce que** le régulateur de pH (7) est au moins partiellement immobilisé et au moins sur une couche parmi la couche de transfert supérieure (15a) ou inférieure (15b), en particulier immobilisé au moyen d'un adhésif.
